# EUROPÄISCHE PATENTSCHRIFT

(11) **EP 2 282 986 B1**
(45) Veröffentlichungstag und Bekanntmachung des Hinweises auf die Patenterteilung: **19.04.2017**
(21) Anmeldenummer: 09753852.4
(22) Anmeldetag: 25.05.2009
(51) Int. Cl.: C07C 51/10, C07C 67/36, C07C 231/00, C07C 231/02, C07C 63/04, C07C 69/76, C07C 235/84, C07C 231/10

(54) **VERFAHREN ZUR HERSTELLUNG VON AROMATISCHEN UND HETEROAROMATISCHEN CARBONSÄUREN, CARBONSÄUREESTERN UND CARBONSÄUREAMIDEN**
METHOD FOR PRODUCING AROMATIC AND HETEROAROMATIC CARBOXYLIC ACIDS, CARBOXYLIC ACID ESTERS AND CARBOXYLIC ACID AMIDES
PROCÉDÉ DE PRODUCTION D'ACIDES CARBOXYLIQUES AROMATIQUES ET HÉTÉROAROMATIQUES, D'ESTERS D'ACIDE CARBOXYLIQUE ET D'AMIDES D'ACIDE CARBOXYLIQUE

(30) Priorität: 27.05.2008 EP 08156993
(43) Veröffentlichungstag der Anmeldung: 16.02.2011
(73) Patentinhaber: BASF SE, 67056 Ludwigshafen am Rhein (DE)
(72) Erfinder: CHALLAND, Nina, 68165 Mannheim (DE); ALTENHOFF, Ansgar, Gereon, 69117 Heidelberg (DE); SCHMIDT-LEITHOFF, Joachim, 68165 Mannheim (DE); WISSEL-STOLL, Kathrin, 67056 Ludwigshafen (DE); RACK, Michael, 69214 Eppelheim (DE); RHEINHEIMER, Joachim, 67063 Ludwigshafen (DE)
(74) Vertreter: BASF IP Association
(86) Internationale Anmeldenummer: PCT/EP2009/056302
(87) Internationale Veröffentlichungsnummer: WO 2009/144197

(56) Entgegenhaltungen:
- EP-A- 0 282 266
- US-A- 5 344 961
- MANSOUR, AMAL ET AL: "Efficient heterogeneously catalyzed amidocarbonylation of bromoarenes based on a serinol-derived chelate diphosphine ligand" JOURNAL OF MOLECULAR CATALYSIS A: CHEMICAL , 250(1-2), 40-43 CODEN: JMCCF2; ISSN: 1381-1169, 2006, XP002545504

## Beschreibung

### Gebiet der Erfindung

Die vorliegende Erfindung betrifft ein neues Verfahren zur Herstellung von aromatischen und heteroaromatischen Carbonsäuren, Carbonsäureestern und Carbonsäureamiden.

### Stand der Technik

Verfahren zur Herstellung von aromatischen und heteroaromatischen Carbonsäuren, Carbonsäureestern und Carbonsäureamiden durch die Umsetzung von aromatischen oder heteroaromatischen Halogeniden der allgemeinen Formel I:

R-Xₙ (I),

worin der Index und die Variablen die folgende Bedeutung haben:
- n: ganze Zahl von 1 bis 4,
- R: substituierter oder unsubstituierter, aromatischer oder heteroaromatischer Rest und
- X: Chlor-, Brom- oder lodatom, insbesondere Chloratom;
mit Kohlenmonoxid und Wasser, Ammoniak, Alkoholen oder Aminen in der Gegenwart von null- oder zweiwertigen Palladiumverbindungen, zweizähnigen Diphosphanen und Basen sind aus dem amerikanischen Patent US 5,344,961 oder der europäischen Patentanmeldung EP 0 282 266 A2 bekannt.

Im amerikanischen Patent US 5,344,961 werden zweizähnige Diphosphane der allgemeinen Formel

(R-)₂P-R'-P(-R)₂

worin die Variablen die folgende Bedeutung haben:
- R: aromatischer Rest mit bis zu 15 Kohlenstoffatomen, wobei mindestens ein aromatischer Ring von R mit einem Rest, der Elektronen anzieht oder einen negativen induktiven Effekt aufweist, insbesondere Alkyloxy- oder Dialkylamino-Gruppen, substituiert ist, und
- R': verknüpfender Kohlenwasserstoffrest mit bis zu 10, vorzugsweise 2 bis 4, Kohlenstoffatomen;
verwendet. Bevorzugte Reste R sind 2-Methoxyphenyl, 2-Propoxyphenyl, 2,4-Diethoxyphenyl, 2-Dimethylaminophenyl, 2-Ethymethylaminophenyl und 2,4,6-Trimethoxyphenyl, insbesondere 2-Methoxyphenyl. Geeignete Reste R' sind 1,2-Ethylen, 1,3-Propylen, 1,4-Butylen, 2,2-Dimethyl-1,3-propylen und 2,3-Dimethyl-1,4-butylen. Bevorzugt wird indes 1,3-[Bis(2-methoxyphenyl)phosphanyl]propan verwendet. Wie aus den Beispielen des amerikanischen Patents hervorgeht, konnte lediglich Brombenzol mit Kohlenmonoxid und Alkohol in der Gegenwart von Palladiumacetat und 1,3-[Bis(2-methoxyphenyl)phosphanyl]propan quantitativ zu Benzoesäureethylester umgesetzt werden, wogegen Chlorbenzol nur zu etwa 10% umgesetzt wurde. 4-Methoxychlorbenzol wurde sogar nur zu 5% zu 4-Methoxybenzoesäureethylester umgesetzt. Wurde 1,3-[Bis(2-methoxyphenyl)phosphanyl]propan durch 1,3-Bis(diphenylphosphanyl)propan ersetzt, lieferte Brombenzol selbst nach 15 Stunden nur Spuren von Benzoesäureethylester.

In der europäischen Patentanmeldung EP 0 282 266 A2 werden eine Reihe von Diphosphanen zur Verwendung in dem oben genannten Verfahren vorgeschlagen. In den Beispielen 1 bis 21 wird aber ausschließlich 1,4-Bis(diphenylphosphanyl)butan verwendet. Die Ausbeute an aromatischen Estern und Amiden ist zwar höher als bei dem Verfahren gemäß dem amerikanischen Patent US 5,344,961, es werden aber auch hier nur niedrige bis mittlere Ausbeuten erzielt.

Deswegen kann der Fachmann weder der europäischen Patentanmeldung EP 0 282 266 A2 noch dem amerikanischen Patent US 5,344,961 Anregungen oder Hinweise darauf entnehmen, wie die Ausbeute an Wertprodukten auch im Falle der aromatischen Chloride signifikant erhöht werden könnte.

In der älteren europäischen Patentanmeldung mit der Anmeldenummer EP 07109463.5 vom 1. Juni 2007 ist die Umsetzung von 4-Brom-3-difluormethyl-1-methylpyrazol mit 2-(3,4,5-Trifluorphenyl)anilin und Kohlenmonoxid zu N-[2-(3,4,5-Trifluorphenyl)phenyl]-3-difluormethyl-1-methylpyrazol-4-carbonsäureamid
(a) in N-Methylpyrrolidon in der Gegenwart von Pd(C₆H₅CN)₂Cl₂, 2,2-Dimethyl-1,3-bis(diphenylphosphanyl)propan und Kaliumcarbonat oder
(b) in Acetonitril in der Gegenwart von Pd(C₆H₅CN)₂Cl₂, 3,3-Bis(diphenylphosphanylmethylen)heptan, Triethylamin und Kaliumcarbonat
beschrieben. In der älteren europäischen Patentanmeldung wird 2,2-Dimethyl-1,3-bis(diphenylphosphanyl)propan als Pepstar und Bis(diphenylphosphanylmethylen)heptan als Et,Bu-Pepstar bezeichnet.

### Aufgabe

Der vorliegenden Erfindung lag die Aufgabe zu Grunde, ein neues, alternatives Verfahren der eingangs genannten Art bereitzustellen, dass nicht nur im Falle von aromatischen Bromiden, sondern auch im Falle von aromatischen und heteroaromatischen Chloriden aromatische und heteroaromatische Carbonsäureester und Carbonsäureamide in besonders hohen, insbesondere nahezu quantitativen oder quantitativen, Ausbeuten liefert. Außerdem soll es das neue Verfahren ermöglichen, auch im Falle von aromatischen und heteroaromatischen Chloriden, aromatische und heteroaromatische Carbonsäuren in besonders hoher, insbesondere nahezu quantitativer oder quantitativer, Ausbeute herzustellen.

### Erfindungsgemäße Lösung

Demgemäß wurde das neue Verfahren zur Herstellung von aromatischen und heteroaromatischen Carbonsäuren, Carbonsäureestern und Carbonsäureamiden durch die Umsetzung von aromatischen oder heteroaromatischen Halogeniden der allgemeinen Formel I:

R-Xₙ (I),

worin der Index und die Variablen die folgende Bedeutung haben:
- n: ganze Zahl von 1 bis 6,
- R: substituierter oder unsubstituierter, aromatischer oder heteroaromatischer Rest und
- X: Chlor-, Brom- oder lodatom;
mit Kohlenmonoxid und Wasser, Ammoniak, Alkoholen oder Aminen in der Gegenwart von Basen und null- oder zweiwertigen Palladiumverbindungen und zweizähnigen Diphosphanen oder Komplexen von null- oder zweiwertigem Palladium mit zweizähnigen Diphosphanen, bei dem man zweizähnige Diphosphane der allgemeinen Formel II verwendet:

(R¹-)(R²-)P-Y-P(-R³)(-R⁴) (II),

worin die Variablen die folgende Bedeutung haben:
- R¹ bis R⁴: unabhängig voneinander gleich oder verschieden, unsubstituierte Arylreste oder unsubstituierte oder substituierte Cycloalkylreste; und
- Y: Kohlenwasserstoffgruppe mit insgesamt 2 bis 20 Kohlenstoffatomen, wobei mindestens eines der Kohlenstoffatome nur ein oder kein Wasserstoffatom als Substituent trägt;
gefunden, ausgenommen die Umsetzung von 4-Brom-3-difluormethyl-1-methylpyrazol mit 2-(3,4,5-Trifluorphenyl)anilin und Kohlenmonoxid zu N-[2-(3,4,5-Trifluorphenyl)phenyl]-3-difluormethyl-1-methylpyrazol-4-carbonsäureamid
(a) in N-Methylpyrrolidon in der Gegenwart von Pd(C₆H₅CN)₂Cl₂, 2,2-Dimethyl-1,3-bis(diphenylphosphanyl)propan und Kaliumcarbonat oder
(b) in Acetonitril in der Gegenwart von Pd(C₆H₅CN)₂Cl₂, 3,3-Bis(diphenylphosphanylmethylen)heptan, Triethylamin und Kaliumcarbonat.

Im Folgenden wird das neue Verfahren zur Herstellung von aromatischen und heteroaromatischen Carbonsäuren, Carbonsäureestern und Carbonsäureamiden als »erfindungsgemäßes Verfahren« bezeichnet.

### Vorteile der Erfindung

Im Hinblick auf den Stand der Technik war es überraschend und für den Fachmann nicht vorhersehbar, dass die Aufgabe, die der vorliegenden Erfindung zu Grunde lag, mithilfe des erfindungsgemäßen Verfahrens gelöst werden konnte.

Insbesondere war es überraschend, dass das erfindungsgemäße Verfahren nicht nur im Falle von aromatischen Bromiden, sondern auch im Falle von aromatischen und heteroaromatischen Chloriden aromatische und heteroaromatische Carbonsäureester und Carbonsäureamide in besonders hohen, insbesondere nahezu quantitativen oder quantitativen, Ausbeuten lieferte. Außerdem ermöglichte es das neue Verfahren, auch im Falle von aromatischen und heteroaromatischen Chloriden, aromatische und heteroaromatische Carbonsäuren in besonders hoher, insbesondere nahezu quantitativer oder quantitativer, Ausbeute herzustellen.

### Ausführliche Beschreibung der Erfindung

Für das erfindungsgemäße Verfahren ist die Verwendung der zweizähnigen Diphosphane der allgemeinen Formel II:

(R¹-)(R²-)P-Y-P(-R³)(-R⁴)

wesentlich.

Im Rahmen der vorliegenden Erfindung wird für die Bezeichnung der zweizähnigen Diphosphane II die IUPAC-Nomenklatur verwendet (vgl. Römpp Online 2008, »Phosphane«). Außerdem wird im Rahmen der vorliegenden Erfindung für die Bezeichnung der zweizähnigen Diphosphane II der Einfachheit und Klarheit halber die Propan-Kette bzw. der in 2- oder 2,2-Stellung substituierte Prop-1,3-ylen-Rest als Grundstruktur zu Grunde gelegt, auch wenn dies im Einzelfall den IUPAC-Regeln widerspricht.
Im Rahmen der vorliegenden Erfindung werden inerte Substituenten und verknüpfende Gruppen, d.h. Substituenten und verknüpfende Gruppen, die unter den Bedingungen des erfindungsgemäßen Verfahrens mit keinem der hierbei verwendeten Ausgangsprodukte und entstehenden Endprodukte reagieren und/oder Zersetzungsreaktionen initiieren und/oder katalysieren, verwendet. Die inerten Substituenten können aber induktive Effekte und/oder Resonanzeffekte bewirken.
In der allgemeinen Formel II sind die Reste R¹ bis R⁴ unabhängig voneinander gleich oder verschieden und stehen für unsubstituierte Arylreste oder unsubstituierte oder substituierte Cycloalkylreste.

Vorzugsweise weisen die unsubstituierten Arylreste 6 bis 20 Kohlenstoffatome im Ring oder in den Ringen auf.
Bevorzugt werden die Reste R¹ bis R⁴ aus der Gruppe, bestehend aus unsubstituiertem Phenyl und Naphthyl, ausgewählt. Insbesondere wird unsubstituiertes Phenyl verwendet.

Vorzugsweise weisen die unsubstituierten und Cycloalkylreste R¹ bis R⁴ 5 bis 16 Kohlenstoffatome im Ring oder in den Ringen auf. Bevorzugt werden Cyclopentyl und Cyclohexyl, insbesondere wird Cyclohexyl verwendet.

Insbesondere sind die Reste R¹ bis R⁴ unsubstituierte Phenyl- und Cyclohexylreste.
In der allgemeinen Formel II steht die Variable Y für eine Kohlenwasserstoffgruppe mit insgesamt 2 bis 20, vorzugsweise 3 bis 16 und insbesondere 3 bis 10 Kohlenstoffatomen, wobei mindestens eines, vorzugsweise eines, der Kohlenstoffatome nur ein oder kein, vorzugsweise kein, Wasserstoffatom als Substituent trägt.

Die Kohlenwasserstoffgruppen Y enthalten Kohlenstoffatome und Wasserstoffatome oder sie bestehen ausschließlich aus diesen. Vorzugsweise bestehen sie ausschließlich aus Kohlenstoffatomen und Wasserstoffatomen.
Die Kohlenwasserstoffgruppen Y können aromatische Gruppen oder cycloaliphatische Gruppen enthalten.
Die Kohlenwasserstoffgruppen Y können substituiert und unsubstituiert sein.
Beispiele geeigneter Kohlenwasserstoffgruppen Y sind Ethyliden, Propyliden, Butyliden, Cyclopent-1,2- und -1,3-ylen, Cyclopentyliden, Cyclohex-1,2-, -1,3- und -1,4-ylen, Cyclohexyliden oder die in den Strukturen Ya) bis Yv) enthaltenen zweibindigen Kohlenwasserstoffgruppen:

| | | | |
|---|---|---|---|
| a) | 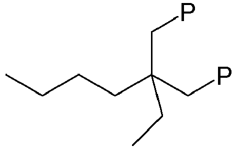 | b) | 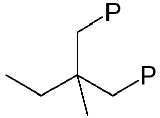 |
| c) | 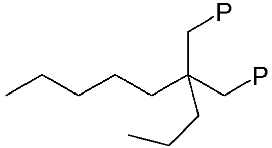 | d) | 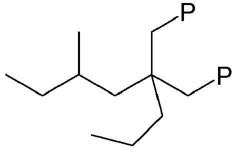 |
| e) | 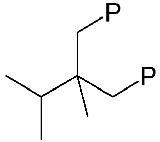 | f) | 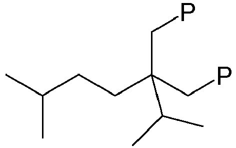 |
| g) | 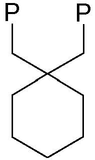 | h) | 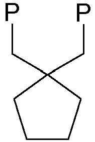 |
| i) | 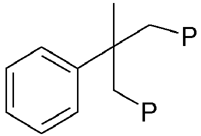 | j) | 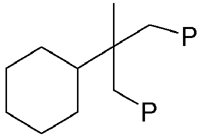 |
| k) | 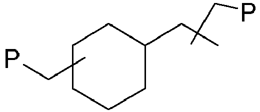 | l) | 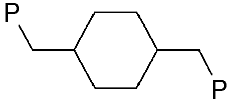 |
| m) | 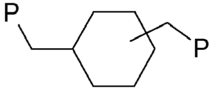 | n) | 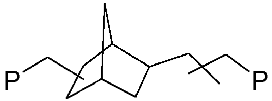 |
| o) | 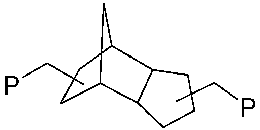 | p) | 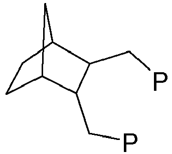 |
| q) | 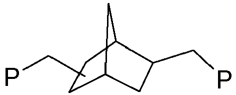 | r) | 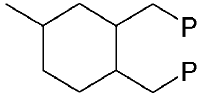 |
| s) | 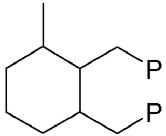 | t) | 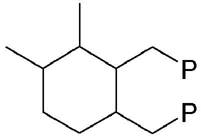 |
| u) | 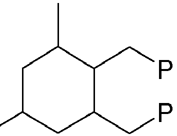 | v) | 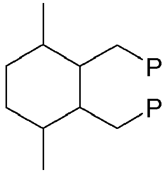 |

Beispiele für Diphosphane II mit chiralen Kohlenwasserstoffgruppen Y, wie (+)-NORPHOS, (R,R)-CHIRAPHOS oder (-)-DIOP, sind aus Thieme Römpp Online 2008, »Phosphane«, bekannt.

Besonders bevorzugt werden Kohlenwasserstoffgruppen Y der allgemeinen Formel III verwendet:

-C(-R⁷)₂-C(-R⁵)(-R⁶)-C(R⁷)₂- (III).

In der allgemeinen Formel III sind die Variablen R⁵ und R⁶ unabhängig voneinander gleich oder verschieden und stehen für Wasserstoffatome, substituierte oder unsubstituierte, lineare oder verzweigte Alkylreste, substituierte oder unsubstituierte Cycloalkylreste oder Arylreste oder Reste, die mindestens zwei dieser Reste enthalten oder hieraus bestehen, wobei nur einer der Reste R⁵ und R⁶ ein Wasserstoffatom ist.

Als Substituenten für die substituierten Reste R⁵ und R⁶ können die vorstehend bei den Resten R¹ und R⁴ beschriebenen Substituenten verwendet werden. Die Substituenten können über kovalente Verbindungen oder die nachstehend beschriebenen inerten zweibindigen verknüpfenden Gruppen mit den Resten R⁵ und R⁶ verbunden sein.

Vorzugsweise ist in einem gegebenen zweizähnigen Diphosphan II keiner der Reste R⁵ oder R⁶ ein Wasserstoffatom.

Sofern es sich bei keinem der Reste der Reste R⁵ oder R⁶ um ein Wasserstoffatom handelt, können sie cyclisch miteinander verknüpft sein. Dabei kann die Verknüpfung über eine Kohlenstoff-Kohlenstoff-Einfachbindung oder eine inerte zweibindige verknüpfende Gruppe, die vorzugsweise aus der Gruppe, bestehend aus Alkylen-, Cycloalkylen-, Arylen-, Ether-, Thioether-, Carbonsäureester-, Thiocarbonsäureester-, Carbonat-, Thiocarbonat-, Phosphorsäureester-, Thiophosphorsäureester-, Phosphonsäureester-, Thiophosphonsäureester-, Phosphit-, Thiophosphit-, Sulfonsäureester-, Amid-, Amin-, Thioamid-, Phosphorsäureamid-, Thiophosphorsäureamid-, Phosphonsäureamid-, Thiophosphonsäureamid-, Sulfonsäureamid-, Imid-, Hydrazid-, Urethan-, Harnstoff-, Thioharnstoff-, Carbonyl-, Thiocarbonyl-, Sulfon- oder Sulfoxidgruppen, ausgewählt wird, erfolgen.

### Vorzugsweise werden die Reste R⁵ und R⁶ aus der Gruppe, bestehend aus

- substituierten und unsubstituierten, vorzugsweise unsubstituierten, linearen oder verzweigten Alkylresten mit einem Kohlenstoffatom oder 2 bis 12 Kohlenstoffatomen, insbesondere unsubstituierten Alkylresten mit einem Kohlenstoffatom oder 2 bis 6 Kohlenstoffatomen;
- substituierten und unsubstituierten Cycloalkylresten mit 5 bis 16 Kohlenstoffatomen im Ring oder in den Ringen, insbesondere unsubstituierten und mit Alkylresten, vorzugsweise Alkylresten mit 1 bis 4 Kohlenstoffatomen, substituierten Cycloalkylresten mit 5 bis 16 Kohlenstoffatomen im Ring oder in den Ringen;
- substituierten und unsubstituierten Arylresten mit 6 bis 20 Kohlenstoffatomen im Ring oder in den Ringen, insbesondere unsubstituierten und mit Alkylresten, vorzugsweise Alkylresten mit 1 bis 4 Kohlenstoffatomen, substituierten Arylresten mit 6 bis 20 Kohlenstoffatomen im Ring oder in den Ringen;
- substituierten und unsubstituierten x-Cycloalkylalkan-1-yl-Resten mit 5 bis 16 Kohlenstoffatomen im Cycloalkylrest, insbesondere unsubstituierten und mit Alkylresten, vorzugsweise Alkylresten mit 1 bis 4 Kohlenstoffatomen, substituierten Cycloalkylresten mit 5 bis 16 Kohlenstoffatomen im Cycloalkylrest, sowie mit jeweils einem Kohlenstoffatom oder 2 bis 6 Kohlenstoffatomen im 1,x-Alkylen-Rest der x-Cycloalkylalkan-1-yl-Reste, wobei x = ganze Zahl von 1 bis 6, vorzugsweise 1 bis 4;
- substituierten und unsubstituierten x-Arylalkan-1-yl-Resten mit 6 bis 20 Kohlenstoffatomen im Arylrest, insbesondere unsubstituierten und mit Alkylresten, vorzugsweise Alkylresten mit 1 bis 4 Kohlenstoffatomen, substituierten Arylresten mit 6 bis 20 Kohlenstoffatomen im Arylrest, sowie mit jeweils einem Kohlenstoffatom oder 2 bis 6 Kohlenstoffatomen im 1,x-Alkylen-Rest der x-Arylalkan-1-yl-Reste, wobei x = ganze Zahl von 1 bis 6, vorzugsweise 1 bis 4; oder
- substituierten und unsubstituierten y-Arylcycloalkan-1-yl-Resten mit 6 bis 20 Kohlenstoffatomen im Arylrest und 5 bis 16 Kohlenstoffatomen im 1,y-Cycloalkylen-Rest der y-Arylcycloalkan-1-yl-Reste, insbesondere unsubstituierten und mit Alkylresten, vorzugsweise Alkylresten mit 1 bis 4 Kohlenstoffatomen, substituierten Arylresten mit 6 bis 20 Kohlenstoffatomen im Arylrest und Cycloalkylresten mit 5 bis 16 Kohlenstoffatomen im Cycloalkylrest, wobei y = ganze Zahl von 1 bis 12, vorzugsweise 1 bis 4;
ausgewählt.

Bevorzugt werden die linearen oder verzweigten Alkylreste R⁵ und R⁶ aus der Gruppe, bestehend aus Methyl, Ethyl, Propyl, Isopropyl, n-Butyl, sec-Butyl, tert-Butyl, n-Pentyl, Isopentyl, Neopentyl, n-Hexyl, n-Heptyl, n-Octyl, 2,2-Dimethylhexyl, 3-Methyl-2-ethyl-pentyl, Nonyl, Decyl, 5-Methyl-nonyl, Undecyl und Dodecyl, ausgewählt.

Bevorzugt werden die Cycloalkylreste R⁵ und R⁶ aus der Gruppe, bestehend aus Cyclopentyl und Cyclohexyl sowie den Resten, die sich von Norcaran, Norpinan, Norbornan, Bornan, 10-Norbornan, o-Menthan, m-Menthan, p-Menthan, Thujan, Caran, Pinan, 2-Ethyl-pinan, 2,4,7,7-Tetramethylnorcaran und 2,2-Dimethylnorbornan ableiten und über ein Ring-Kohlenstoffatom mit der Grundstruktur der allgemeinen Formel II, d. h. mit der 2- oder 2,2-Stellung des Prop-1,3-ylen-Rests, verknüpft sind, ausgewählt.

Bevorzugt werden die x-Cycloalkylalkan-1-yl-Reste R⁵ und R⁶ aus der Gruppe, bestehend aus Cyclohexylmethyl, 2-Cyclohexyleth-1-yl, 3-Cyclohexyl-prop-1-yl und 4-Cyclohexyl-but-1-yl sowie den Resten, die sich von Bornan, 10-Norbornan, o-Menthan, m-Menthan, p-Menthan, Thujan, Caran, Pinan, 2-Ethyl-pinan, 2,4,7,7-Tetramethylnorcaran und 2,2-Dimethylnorbornan ableiten und über ein nicht im Ring befindliches aliphatisches Kohlenstoffatom mit der Grundstruktur der allgemeinen Formel II verknüpft sind, ausgewählt.

Bevorzugt werden die Arylreste R⁵ und R⁶ aus der Gruppe, bestehend aus Phenyl und Naphthyl sowie den Resten, die sich von Toluol, Xylol, Propylbenzol, Isopropylbenzol, n-Butylbenzol, sec-Butylbenzol und tert-Butylbenzol ableiten und über ein aromatisches Ring-Kohlenstoffatom mit der Grundstruktur der allgemeinen Formel II verknüpft sind, ausgewählt.

Bevorzugt werden die x-Arylalkan-1-yl-Reste R⁵ und R⁶ aus der Gruppe, bestehend aus den Resten, die sich von Toluol, Xylol, Propylbenzol, Isopropylbenzol, n-Butylbenzol, sec-Butylbenzol und tert-Butylbenzol ableiten und über ein nicht im Ring befindliches aliphatisches Kohlenstoffatom mit der Grundstruktur der allgemeinen Formel II verknüpft sind, ausgewählt.

Bevorzugt werden die y-Arylcycloalkan-1-yl-Reste R⁵ und R⁶ aus der Gruppe, bestehend aus den Resten, die sich von Phenylcyclopentan, Phenylcyclohexan, Tolylcyclohexan und Xylylcyclohexan ableiten und über ein im Cycloalkanring befindliches Kohlenstoffatom mit der Grundstruktur der allgemeinen Formel II verknüpft sind, ausgewählt.

Besonders bevorzugt werden die Reste R⁵ und R⁶ aus der Gruppe, bestehend aus Methyl, Ethyl, Propyl, n-Butyl, n-Pentyl, n-Hexyl, Benzyl, Cyclopentyl, Cyclohexyl und Phenyl, ganz besonders bevorzugt Methyl, Ethyl, Propyl, n-Butyl, n-Pentyl, n-Hexyl, Cyclohexyl und Phenyl, insbesondere Methyl, Ethyl und n-Butyl, ausgewählt.

Vorzugsweise werden unsubstituierte Reste R⁵ und R⁶ verwendet.

In der allgemeinen Formel III wird die Variable R⁷ aus der Gruppe, bestehend aus Wasserstoffatomen, Fluoratomen, Chloratomen, Bromatomen, Nitrilgruppen, Nitrogruppen und Resten R⁵ und R⁶ ausgewählt.

Handelt es sich bei den Resten R⁷ um Reste R⁵ und R⁶ können sie über kovalente Verbindungen oder die vorstehend beschriebenen inerten zweibindigen verknüpfenden Gruppen mit den Kohlenstoffatomen der Grundstruktur der allgemeinen Formel III, d. h. dem Propan-1,3-ylen-Rest, verbunden sein.

Die Kohlenwasserstoffgruppe Y der allgemeinen Formel III kann 1 bis 4 Reste R⁷ enthalten. Dabei können mindestens zwei der Reste R⁷ gleich oder voneinander verschieden sein. Vorzugsweise handelt es sich bei einem, vorzugsweise bei zwei und bevorzugt bei drei der Reste R⁷ und insbesondere bei allen vier Resten R⁷ um ein Wasserstoffatom oder Wasserstoffatome.

Bevorzugt werden die zweizähnigen Diphosphane der allgemeinen Formel II aus der Gruppe, bestehend aus
- 2-Methyl-, 2-Ethyl-, 2-Propyl-, 2-n-Butyl-, 2-n-Pentyl-, 2-n-Hexyl-, 2-Cyclohexyl-und 2-Phenyl-1,3-bis(dihenylphosphanyl)propan und -1,3-bis(dicyclohexylphosphanyl)propan,
- 2,2-Dimethyl-, 2,2-Diethyl-, 2,2-Dipropyl-, 2,2-Di-n-butyl-, 2,2-Di-n-pentyl-, 2,2-Di-n-hexyl-, 2,2-Dicyclohexyl-, 2,2-Diphenyl-1,3-bis(diphenylphosphanyl)propan und-1,3-bis(dicyclohexylphosphanyl)propan,
- 2-Methyl-2-ethyl-, -2-propyl-, -2-n-butyl-, -2-n-pentyl-, -2-n-hexyl-, -2-cyclohexyl-und -2-phenyl-1,3-bis(diphenylphosphanyl)propan und -1,3-bis(dicyclohexylphosphanyl)propan,
- 2-Ethyl-2-propyl-, -2-n-butyl-, -2-n-pentyl-, -2-n-hexyl-, -2-cyclohexyl- und -2-phenyl-1,3-bis(diphenylphosphanyl)propan und -1,3-bis(dicyclohexylphosphanyl)propan,
- 2-Propyl-2-n-butyl-, -2-n-pentyl-, -2-n-hexyl-, -2-cyclohexyl- und -2-phenyl-1,3-bis(diphenylphosphanyl)propan und -1,3-bis(dicyclohexylphosphanyl)propan,
- 2-n-Butyl-2-n-pentyl-, -2-n-hexyl-, -2-cyclohexyl-1,3- und -2-phenyl-1,3-bis(diphenylphosphanyl)propan und -1,3-bis(dicyclohexylphosphanyl)propan,
- 2-n-Pentyl-2-n-hexyl-, -2-cyclohexyl-1,3- und -2-phenyl-1,3-bis(diphenylphosphanyl)propan und -1,3-bis(dicyclohexylphosphanyl)propan,
- 2-n-Hexyl-2-cyclohexyl- und -2-phenyl-1,3-bis(diphenylphosphanyl)propan und-1,3-bis(dicyclohexylphosphanyl)propan und
- 2-Cyclohexyl-2-phenyl-1,3-bis(diphenylphosphanyl)propan und -1,3-bis(dicyclohexylphosphanyl)propan,
ausgewählt. Insbesondere werden 2-Ethyl-2-butyl- oder 2,2-Dimethyl-1,3-bis(diphenylphosphanyl)propan oder Gemische hiervon verwendet. 2,2-Dimethyl-1,3-bis(diphenylphosphanyl)propan wird im Folgenden als Pepstar und 2-Ethyl-2-butyl-bis(diphenylphosphanyl)propan als Bustar bezeichnet.

Der Herstellung der Diphosphane II erfolgt mithilfe üblicher und bekannter Verfahren der phosphororganischen Chemie, vorzugsweise durch die Umsetzung eines geeigneten Dicycloalkylfluor-, -chlor- oder -bromphosphans oder eines geeigneten Dicycloarylalkoxy-oder -aryloxyphosphans oder eines Dicycloalkylalkoxy- oder -aryloxyphosphans, insbesondere eines geeigneten Dicycloalkylchlor- oder -bromphosphans, wie z.B. Dicyclohexylchlorphosphan oder-bromphosphan, mit einem geeigneten 1,3- Difluor-, 1,3-Dichlor-, 1,3-Bromchlor-, 1,3-Chlorfluor-, 1,3-Bromfluor- oder 1,3-Dibrompropan, insbesondere 1,3-Dichlor-, 1,3-Bromchlor- oder 1,3-Dibrompropan, wie z.B.
- 1,3-Dichlor-, 1,3-Bromchlor- oder 1,3-Dibrom-2-methyl-, -2-ethyl-, -2-propyl-, -2-n-butyl-, -2-n-pentyl-, -2-n-hexyl-, -2-cyclohexyl-, -2-phenyl- propan;
- 1,3-Dichlor-, 1,3-Bromchlor- oder 1,3-Dibrom-2,2-dimethyl-, -2,2-diethyl-, -2,2-dipropyl-, -2,2-di-n-butyl-, -2,2-di-n-pentyl-, -2,2-di-n-hexyl-, -2,2-dicyclohexyl- und -2,2-diphenyl-propan;
- 1,3-Dichlor-, 1,3-Bromchlor- oder 1,3-Dibrom-2-methyl-2-ethyl-, -2-propyl-, -2-n-butyl-, -2-n-pentyl-, -2-n-hexyl-, -2-cyclohexyl- und -2-phenyl-propan;
- 1,3-Dichlor-, 1,3-Bromchlor- oder 1,3-Dibrom-2-ethyl-2-propyl-, -2-n-butyl-, 2-n-pentyl-, -2-n-hexyl-, -2-cyclohexyl- und -2-phenyl-propan;
- 1,3-Dichlor-, 1,3-Bromchlor- oder 1,3-Dibrom-2-propyl-2-n-butyl-, -2-n-pentyl-, 2-n-hexyl-, -2-cyclohexyl-1,3- und -2-phenyl-propan;
- 1,3-Dichlor-, 1,3-Bromchlor- oder 1,3-Dibrom-2-n-butyl-2-n-pentyl-, -2-n-hexyl-, -2-cyclohexyl-propan; 2-n-pentyl-2-n-hexyl-, -2-cyclohexyl-1,3- und -2-phenyl-propan; -2-n-hexyl-2-cyclohexyl- und -2-phenyl-1,3-propan; sowie
- 1,3-Dichlor-, 1,3-Bromchlor- oder 1,3-Dibrom-2-cyclohexyl-2-phenyl-propan;
insbesondere 1,3-Dichlor-2,2-dimethyl-propan, durch Abspaltung der Halogenatome mit metallischem Natrium. Bevorzugt werden dabei die Reaktionsbedingungen angewandt, die in der internationalen Patentanmeldung WO 2006/084878 A1 oder in L. Brandsma et al., "Application of Transition Metal Catalysts in Organic Synthesis", Springer-Verlag, Berlin 1997, Seiten 6 bis 9, beschrieben werden.

Bei dem erfindungsgemäßen Verfahren werden aromatische oder heteroaromatische Halogenide der allgemeinen Formel I umgesetzt.

In der allgemeinen Formel I R(-X)ₙ steht der Index n für eine ganze Zahl von 1 bis 6, vorzugsweise 1 bis 4, bevorzugt 1 bis 3 und insbesondere 1 oder 2.

Die Variable X steht für Chlor-, Brom- oder lodatom, vorzugsweise Chlor- oder Bromatom, insbesondere Chloratom.

Die Variable R steht für einen substituierten oder unsubstituierten, aromatischen oder heteroaromatischen Rest.

Vorzugsweise leiten sich die aromatischen Reste R von Benzol und von polycyclischen aromatischen Kohlenwasserstoffen ab. Die heteroaromatischen Reste R leiten sich vorzugsweise von monocyclischen und polycyclischen aromatischen Heterocyclen ab.

Bevorzugt werden die polycyclischen aromatischen Kohlenwasserstoffe aus der Gruppe, bestehend aus
- Kohlenwasserstoffen, worin mindestens zwei Benzolkerne, mindestens zwei kondensierte polycyclische aromatische Kohlenwasserstoffe oder mindestens ein Benzolkern und mindestens ein kondensierter polycyclischer aromatischer Kohlenwasserstoff über mindestens eine Kohlenstoff-Kohlenstoff-Einfachbindung miteinander verknüpft sind, und
- kondensierten polycyclischen aromatischen Kohlenwasserstoffen,
ausgewählt.

Besonders bevorzugt werden die polycyclischen aromatischen Kohlenwasserstoffe aus der Gruppe, bestehend aus Biphenyl, den isomeren Triphenylenen, Quaterphenylenen, Quinquephenylenen, Phenylnaphthalenen und Binaphthalenen Biphenylen, asymmetrisches und symmetrisches Indacen, Fluoren, Naphthalin, Acenaphthylen, Acenaphthen, Phenanthren, Fluoren, Anthracen, Chrysen, Pyren, Fluoranthen, Benzo[a]anthracen, Benzo[k]fluoranthen, Benzo[b]fluoranthen, Benzo[a]pyren, Dibenzo[a:h]anthracen, Benzo[g:h:i]perylen und Indeno[1,2,3-c:d]pyren, Tetrabenzonaphthalin und Phenanthro[3,4-c]phen, ausgewählt.

Insbesondere leiten sich die aromatischen Reste R von Benzol oder Naphthalin, insbesondere Benzol, ab.

Vorzugsweise enthalten die aromatischen Heterocyclen mindestens ein Heteroatom, ausgewählt aus der Gruppe, bestehend aus Stickstoffatom, Sauerstoffatom und Schwefelatom.

Bevorzugt werden die aromatischen Heterocyclen aus der Gruppe, bestehend aus Pyrrol, Imidazol, Pyrazol, den isomeren Isothiazolen und Isoxazolen, Pyridin, Pyrazin, Pyrimidin, Pyridazin, 1 H-Pyrrolizin, Indolizin, Isoindol, Indol, 1H-Indazol, Purin, 4H-Chinolizin, Isochinolin, Chinolin, Phthalazin, 1,8-Naphthyridin, Chinoxalin, Chinazolin, Cinnolin, Pteridinin, Carbazol, beta-Carbolin, Phenanthridin, Acridin, Perimidin, 1,7-Phenanthrolin, Phenazin, Phenothiazin, Phenoxazin, Thiophen, Benzo[b]thiophen, Naphtho[2,3-b]thiophen, Thianthren, Furan, Isobenzofuran, Phenoxazin, Thiophthen, Thiophanthren, Thianaphthen, Cumaron, Isocumaron, Indoxazen, Anthranil und Piazthiol, ausgewählt.

Der Rest kann mit mindestens einem im vorstehend beschriebenen Sinne inerten Substituenten substituiert sein. Vorzugsweise wird der Substituent aus der Gruppe, bestehend aus Fluoratom, Nitrilgruppe, Nitrogruppe sowie Substituent, der mindestens einen nicht halogenierten, partiell halogenierten oder perhalogenierten, unverzweigten oder verzweigten Alkylrest mit bis zu 16 Kohlenstoffatomen, mindestens einen nicht halogenierten, partiell halogenierten oder perhalogenierten Cycloalkylrest mit bis zu 16 Kohlenstoffatomen und/oder mindestens einen unfluorierten, partiell fluorierten und perfluorierten Arylrest mit 6 bis 20 Kohlenstoffatomen enthält oder hieraus besteht, ausgewählt. Dabei kann der Substituent als solcher über eine Kohlenstoff-Kohlenstoff-Einfachbindung oder eine inerte, zweibindige funktionelle Gruppe mit dem Rest R verbunden sein und/oder die Alkylreste, Cycloalkylreste und/oder Arylreste in einem solchen Substituenten können über mindestens eine Kohlenstoff-Kohlenstoff-Einfachbindung und/oder mindestens eine inerte, zweibindige funktionelle Gruppe miteinander verbunden sein.

Vorzugsweise wird die inerte, zweibindige funktionelle Gruppe aus der Gruppe, bestehend aus Ether-, Thioether-, Carbonsäureester-, Thiocarbonsäureester-, Carbonat-, Thiocarbonat-, Phosphorsäureester-, Thiophosphorsäureester-, Phosphonsäureester-, Thiophosphonsäureester-, Phosphit-, Thiophosphit-, Sulfonsäureester-, Amid-, Amin-, Thioamid-, Phosphorsäureamid-, Thiophosphorsäureamid-, Phosphonsäureamid-, Thiophosphonsäureamid-, Sulfonsäureamid-, Imid-, Hydrazid-, Urethan-, Harnstoff-, Thioharnstoff-, Carbonyl-, Thiocarbonyl-, Sulfon- oder Sulfoxidgruppen, insbesondere Ether-, Carbonsäureester-, Amid- und Carbonylgruppen, ausgewählt.

Beispiele gut geeigneter aromatischer und heteroaromatischer Halogenide der allgemeinen Formel I sind aus der europäischen Patentanmeldung EP 0 282 266 A2, Seite 5, Zeile 40, bis Seite 6, Zeile 5, bekannt. Auf die Passage wird hier ausdrücklich Bezug genommen.

Beispiele besonders gut geeigneter aromatischer und heteroaromatischer Halogenide der allgemeinen Formel I sind Chlorbenzol, Brombenzol, 2-, 3- und 4-Chlor- und - Bromacetophenon, 2-, 3- und 4-Clor- und -Brombenzoesäuremethyl- und -ethylester, 2-, 3- und 4-Chlor- und -Brombenzaldehyd, 2-, 3- und 4-Chlor- und -Bromanisol, 2-, 3- und 4-Chlor- und -Bromtoluol, 2-, 3- und 4-Chlor- und -Brombenzonitril, 2-, 3- und 4-Chlor-und -Bromnitrobenzol, 2-, 3- und 4-Chlor- und -Bromfluorbenzol, 2-, 3- und 4-Chlor- und-Bromtrifluormethylbenzol, 1- und 2-Chlor- und Bromnaphthalin, 1-Chlor- und -Brom-2-, -3-, -4-, -5-, -6-, -7- und -8-methoxynaphthalin, 2-Chlor- und -Brom-1-, -3-, -4-, -5-, -6-, -7- und -8-methoxynaphthalin, 2-, 3- und 4-Chlor- und -Brombiphenyl, 9-Chlor- und - Bromphenanthren, 1,2-, 1,3- und 1,4-Dichlorbenzol, 1,2,3-, 1,2,4- und 1,3,5-Trichlorbenzol, 1,2,3,4-, 1,2,3,5- und 1,2,4,5-Tetrachlorbenzol, 3-, 4- und 5-Chlor- und - Brompyrazol, 2-, 3- und 4-Chlor- und -Brompyridin, 2- und 3-Chlor- und -Bromthiophen, 2,3-, 2,4- 2,5- und 2,6-Dichlor- und -Dibrompyridin, 2,3-, 2,4- und 2,5-Dichor- und -Dibromthiophen, 2,3-, 2,4-, 2,5-, 2,6-, 2,5-, 2,6-, 2,7- und 2,8-Dichlor- und -Dibromchinolin, 3,4-, 3,5-, 3,6-, 3,7- und 3,8-Dichlor- und -Dibromchinolin, 4,5-, 4,6-, 4,7-und 4,8-Dichlor- und -Dibromchinolin, 5,6-, 5,7- und 5,8-Dichlor- und -Dibromchinolin, 6,7- und 6,8-Dichlor- und -Dibromchinolin sowie 7,8-Dichlor- und -Dibromchinolin.

Die vorstehend beschriebenen aromatischen und heteroaromatischen Halogenide der allgemeinen Formel I können in molarem Überschuss sowohl als Ausgangsprodukte als auch als Lösemittel eingesetzt werden, sofern sie unter den angewandten Reaktionsbedingungen flüssig sind.

Bei dem erfindungsgemäßen Verfahren werden die vorstehend beschriebenen aromatischen und heteroaromatischen Halogenide der allgemeinen Formel I mit Wasser zu Carbonsäuren, mit Ammoniak zu primären Amiden, mit Alkoholen zu Carbonsäureestern und mit Aminen zu sekundären und tertiären Amiden umgesetzt.

Vorzugsweise wird der Alkohol aus der Gruppe, bestehend aus aliphatischen, cycloaliphatischen, aromatischen und heteroaromatischen Alkoholen mit 1 bis 4 Hydroxylgruppen im Molekül, ausgewählt.

Bevorzugt wird der aliphatische Alkohol aus der Gruppe, bestehend aus Methanol, Ethanol, Propanol, Isopropanol, n-Butanol, sec-Butanol, tert-Butanol, n-Amylalkohol, 2-Methyl-2-butanol, n-Hexanol, den isomeren Heptanolen, 4-Methyl-3-heptanol, den isomeren Caprylalkoholen, Nonanolen und Decanolen, Benzylalkohol, 2-Phenylethanol, 3-Phenylpropanol, 4-Phenylbutanol Ethylenglykol, Propylenglykol, den isomeren Butandiolen, Polyethylenglykolen, Polypropylenglykolen und Diethyloctandiolen, N-Phenyldiethanolamin, Glycerin, Trimethylolethan, Trimethylolpropan, Triethanolamin, Erythritol, Threitol und Pentaerythrit, ausgewählt.

Bevorzugt wird der cycloaliphatische Alkohol aus der Gruppe, bestehend aus Cyclopentanol, Cyclohexanol, Borneol, Isoborneol, 1,1-, 1,2-, 1,3- und 1,4-Cyclohexandiol, cis- und trans-1,8-Terpin und hydriertem Bisphenol A und F, ausgewählt.

Bevorzugt wird der aromatische Alkohol aus der Gruppe, bestehend aus Phenol, Brenzkatechin, Resorcin, Hydrochinon, Pyrogallol, Phloroglucin, alpha- und beta-Naphthol sowie Bisphenol A und F, ausgewählt.

Bevorzugt wird der heteroaromatische Alkohol aus der Gruppe, bestehend aus den isomeren Hydroxypyridinen, Hydroxypyrazinen, Hydroxypyrimidinen, Hydroxypyridazinen, 1H-Hydroxypyrrolizinen, 4H-Hydroxychinolizinen, Hydroxyisochinolinen, Hydroxychinolinen, Hydroxyphthalazinen, Hydroxy-1,8-naphthyridinen, Hydroxychinoxalinen, Hydroxychinazolinen, Hydroxycinnolinen, Hydroxypteridinen, Hydroxyphenanthridinen, Hydroxyacridinen und Hydroxy-1,7-phenanthrolinen sowie Allupurinol; ausgewählt.

Die vorstehend beschriebenen Alkohole können als solche oder in der Form von Alkoholaten, in der sie zugleich die Funktion der Base übernehmen, eingesetzt werden.

Vorzugsweise wird das Amin aus der Gruppe, bestehend aus aliphatischen, cycloaliphatischen, cyclischen, aromatischen und heteroaromatischen, primären und sekundären Aminen mit 1 bis 4 Aminogruppen im Molekül, ausgewählt.

Bevorzugt wird das aliphatische oder cycloaliphatische, primäre oder sekundäre Amin aus der Gruppe, bestehend aus Methylamin, Ethylamin, Propylamin, Isopropylamin, den isomeren Butylaminen, Pentylaminen, Hexylaminen, Heptylaminen, Octylaminen, Nonylaminen und Decylaminen, Dodecylamin, Benzylamin, den Phenylethanaminen, Hydrazin, 1,2-Ethylendiamin, 1,3-Diaminopropan, Putrescin, Cadaverin, 1,6-Diaminohexan, Diethylentriamin, Triethylentetramin, Spermin, Cyclohexylamin und Dicyclohexylamin, ausgewählt.

Bevorzugt wird das cyclische, sekundäre Amin aus der Gruppe, bestehend aus Pyrrol, Imidazol, Pyrazol, Isoindol, Pyrrolidin, Piperidin, Morpholin, Isoindolin Imidazolidin, Pyrazolidin und Piperazin, ausgewählt.

Bevorzugt werden die aromatischen und heteroaromatischen, primären und sekundären Amine aus der Gruppe, bestehend aus Anilin, den isomeren Phenylendiaminen, Diphenylamin, den isomeren Biphenyldiaminen, Biphenyltriaminen und Biphenyltetraminen, Indol, Indolin, 1H-Indazol, Purin, Carbazol, beta-Carbolin, Perimidin, Phenothiazin, den isomeren Aminopyridinen, Aminopyrazinen, Aminopyrimidinen, Aminopyridazinen, 1 H-Aminopyrrolizinen, 4H-Aminochinolizinen, Aminoisochinolinen, Aminochinolinen, Aminophthalazinen, Amino-1,8-naphthyridinen, Aminochinoxalinen, Aminochinazolinen, Aminocinnolinen, Aminopteridinen, Aminophenanthridinen, Aminoacridinen und Amino-1,7-phenanthrolinen, Benzoguanamin und Melamin, ausgewählt.

Die vorstehend beschriebenen Amine können in molarem Überschuss sowohl als Ausgangsprodukte als auch als Basen eingesetzt werden.

Der jeweilige Grundkörper der Alkohole und/oder der Amine kann mit mindestens einem inerten Substituenten substituiert sein. Vorzugsweise wird der inerte Substituent aus der Gruppe, bestehend aus den vorstehend beschriebenen Substituenten, ausgewählt.

Das erfindungsgemäße Verfahren wird in der Gegenwart von mindestens einer null- oder zweiwertigen Palladiumverbindung durchgeführt.

Vorzugsweise wird die nullwertige Palladiumverbindung aus der Gruppe, bestehend aus metallischem Palladium und Palladium(0)-organylen und die zweiwertige Palladiumverbindung aus der Gruppe, bestehend aus Palladium(II)-organylen und Palladium(II)-salzen, ausgewählt.

Bevorzugt ist das metallische Palladium geträgert. Beispiele geeigneter, im vorstehend beschriebenen Sinne inerter Trägermaterialien sind Aktivkohle, Aluminiumoxid und Aluminate, Siliciumdioxid und Silikate, Bariumsulfat und Calciumcarbonat. Bei der Verwendung von metallischem Palladium werden bevorzugt noch übliche und bekannte Liganden, die in der Lage sind Palladium(0) zu komplexieren, eingesetzt.

Bevorzugt wird das Palladium(0)-organyl aus der Gruppe, bestehend aus Tris(η²-alken)-palladium(0), Bis(carben)-palladium(0), Palladium(0)-Phosphankomplexen und gemischten Palladium(0)-(η²-Alken)-Phosphankomplexen, ausgewählt.

Bevorzugt wird das Palladium(II)-organyl aus der Gruppe, bestehend aus Palladium(II)-Chelatkomplexen, Donoraddukten mit einwertigen Liganden oder Komplexen mit einwertigen Liganden und Halogeniden und pi-gebundenen Liganden, ausgewählt.

Bevorzugt wird das Palladium(II)-salz aus der Gruppe, bestehend aus Palladium(II)-halogeniden und -carboxylaten, ausgewählt.

Beispiele geeigneter nullwertiger und zweiwertiger Palladiumverbindungen sind aus Hollemann-Wiberg, Lehrbuch der Anorganischen Chemie, Nils Wiberg, 102. Auflage, 2007, »2.2 Verbindungen des Palladiums und Platins«, Seiten 1726 bis 1743, insbesondere »2.2.6 Organische Verbindungen des Palladiums und Platins«, Seiten 1739 bis 1734, bekannt. Auf diese Passagen wird hier ausdrücklich Bezug genommen.

Bevorzugt werden Palladium(II)-chlorid, Palladium(II)-acetat, Palladium(II)-acetylacetonat, Dichlor-bis(cyanophenyl)-palladium(II), Dichlor-bis(diphenylphosphanyl)-palladium(II) und/oder Tetrakis(diphenylphosphanyl)-palladium(II), insbesondere Palladium(II)-acetat, verwendet.

Die Palladiumverbindungen können als solche bei der erfindungsgemäßen Umsetzung eingesetzt werden. Es ist aber auch möglich, sie zuvor mit den Diphosphanen II in an sich bekannter Weise zu Palladium(0)- oder Palladium(II)-Diphosphan-Komplexen umzusetzen. Die resultierenden Komplexe können dann in der erfindungsgemäßen Umsetzung verwendet werden. Beispiele sehr gut geeigneter Komplexe sind
- Pd[2,2-dimethyl-1,3-bis(diphenylphosphanyl)propan]₂Cl₂ oder Pd(Pepstar)₂Cl₂ und
- Pd[2-ethyl-2-butyl-bis(diphenylphosphanyl)propan]₂Cl₂ oder Pd(Bustar)₂Cl₂.

Die erfindungsgemäße Umsetzung wird in der Gegenwart mindestens einer, insbesondere einer, Base durchgeführt.

Vorzugsweise wird die Base aus der Gruppe, bestehend aus Alkalimetallsalzen, Alkoholaten, den vorstehend beschriebenen überschüssigen Aminen und tertiären Aminen, ausgewählt.

Beispiele geeigneter Alkalimetallsalze sind Natriumphosphat, Kaliumphosphat, Natriumarbonat, Kaliumcarbonat, Natriumacetat und Kaliumacetat. Bei der Herstellung der Carbonsäureester und der Carbonsäurenamide nach dem erfindungsgemäßen Verfahren werden sie bevorzugt wasserfrei eingesetzt. Insbesondere wird wasserfreies Kaliumcarbonat verwendet.

Beispiele geeigneter Alkoholate sind die Natrium- und Kaliumalkoholate der vorstehend beschriebenen Alkohole, insbesondere Natrium- und Kaliummethanolat, -ethanolat, - isopropylat, -tert-butylat und -phenolat.

Beispiele geeigneter tertiärer Amine sind Trimethylamin, Triethylamin, Diisopropylethylamin, N-Methylpiperidin, Pyridin, Collidin, Lutidin, 4-Dimethylaminopyridin, 1,4-Diazabicyclo[2.2.2]octan (DABCO), 1,5-Diazabicyclo[4.3.0]non-5-en (DBN) und 1,8-Diazabicyclo[5.4.0]undec-7-en (DBU), insbesondere Triethylamin und DBU.

Bei der Umsetzung gemäß dem erfindungsgemäßen Verfahren werden die aromatischen oder heteroaromatischen Halogenide der allgemeinen Formel I mit Wasser, Alkoholen oder Aminen vorzugsweise in einem Molverhältnis von Halogenid I : Wasser, Alkohol oder Amin von 0,5 : 1 bis 2 : 1, insbesondere 0,8 : 1 bis 1,2 : 1, jeweils bezogen auf das jeweilige nucleophile Sauerstoffatom oder Stickstoffatom, umgesetzt.

Bevorzugt wird dabei die nullwertige oder zweiwertige Palladiumverbindung in einer Menge von 0,001 bis 5 Mol-%, insbesondere 0,01 bis 1 Mol-%, jeweils bezogen auf das Halogenid I, eingesetzt.

Besonders bevorzugt wird das zweizähnige Diphosphan II in einem Molverhältnis von zweizähniges Diphosphan I zu Palladiumverbindung von 0,01 : 1 bis 10.000 : 1, insbesondere 0,1 : 1 bis 100 : 1, verwendet.

Bevorzugt wird die Base in einem Äquivalentverhältnis von Halogenatom im Halogenid I zu Base = 1 : 1 bis 1 : 4 verwendet. Bei der Verwendung eines Alkalimetallsalzes wird besonders bevorzugt ein Äquivalentverhältnis von 1 : 1 bis 1 : 4, insbesondere 1 : 2, angewandt. Bei der Verwendung eines tertiären Amins wird besonders bevorzugt ein Äquivalentverhältnis von 1 : 0,1 bis 1 : 4, insbesondere 1 : 0,2 bis 1 : 2 angewandt.

Die Umsetzung nach dem erfindungsgemäßen Verfahren kann in Abwesenheit eines organischen Lösemittels durchgeführt werden. Sie kann aber auch in einem aromatischen oder einem aprotisch polaren organischen Lösemittel durchgeführt werden.

Vorzugsweise werden die aromatischen Lösemittel aus der Gruppe, bestehend aus überschüssigen aromatischen oder heteroaromatischen Halogeniden der allgemeinen Formel I, Toluol und den Xylolen, ausgewählt.

Vorzugsweise werden die aprotisch polaren organischen Lösemittel aus der Gruppe, bestehend aus Amiden, Ethern, Sulfonen und Nitrilen, ausgewählt. Bevorzugt werden Dimethylformamid, Dimethylacetamid, N-Methylpyrrolidon, Hexamethylphosphorsäuretriamid, 1,4-Dioxan, Sulfolan, Acetonitril, Propionitril sowie Gemische hiervon verwendet.

Besonders bevorzugt ist das organische Lösemittel bei der Umsetzung der Halogenide I mit Alkoholen oder Aminen im Wesentlichen oder völlig wasserfrei. Dies bedeutet, dass es einen Wassergehalt von < 1.000 ppm, insbesondere < 100 ppm aufweist.

Die erfindungsgemäße Umsetzung kann außerdem in der Gegenwart eines organischen Monophosphans durchgeführt werden. Die Monophosphane enthalten mindestens einen Alkylrest, Cycloalkylrest oder Arylrest. Bevorzugt enthalten die Monophosphane zwei und insbesondere drei Reste, ausgewählt aus der Gruppe, bestehend aus Alkylresten, Cycloalkylresten oder Arylresten. Diese Reste können auch cyclisch miteinander verknüpft sein. Insbesondere wird Triphenylphosphan verwendet.

Bevorzugt werden die vorstehend beschriebenen Palladiumkomplexe der Diphosphane II in Kombination mit mindestens einem, insbesondere einem, Monophosphan verwendet.

Vorzugsweise wird die Umsetzung nach dem erfindungsgemäßen Verfahren bei einer Temperatur zwischen 90 und 200°C, bevorzugt 100 bis 180°C und insbesondere 110 bis 150°C, durchgeführt.

Die Umsetzung nach dem erfindungsgemäßen Verfahren kann bei leichtem Unterdruck, Normaldruck oder Überdruck durchgeführt werden. Dabei wird der angewandte Druck vor allem durch den Kohlenmonoxid-Partialdruck bestimmt. Bevorzugt wird die Umsetzung bei einem Kohlenmonoxid-Partialdruck von 0,9 bis 100 bar (90 bis 10.000 kPa), besonders bevorzugt 1 bis 50 bar (100 bis 5.000 kPa), insbesondere 5 bis 20 bar (500 bis 2.000 kPa), durchgeführt.

Die Aufarbeitung der nach dem erfindungsgemäßen Verfahren erhaltenen Reaktionsgemische erfolgt vorzugsweise wässrig. Dabei werden die Reaktionsgemische mit Wasser oder einer wässrigen Lösung in Kontakt gebracht. Nach dem Ansäuern der wasserhaltigen Reaktionsgemische können die in der erfindungsgemäßen Verfahrensweise hergestellten Carbonsäuren, Carbonsäureester und Carbonsäureamide durch Extraktion mit organischen Lösemitteln und anschließendem Entfernen der organischen Lösemittel isoliert werden. Gegebenenfalls kann es von Vorteil sein, insbesondere wenn bei der Umsetzung nach dem erfindungsgemäßen Verfahren wassermischbare Lösemittel verwendet wurden, die Lösemittel vor der Extraktion zumindest teilweise, beispielsweise durch Destillation, zu entfernen.

Das erfindungsgemäße Verfahren und die Aufarbeitung der erhaltenen Reaktionsgemische bieten keine methodischen Besonderheiten, sondern können mithilfe der üblichen und bekannten Verfahren und Anlagen der organischen Chemie durchgeführt werden.

### Ausführungsformen

### Ausführungsform 1:

Verfahren zur Herstellung von aromatischen und heteroaromatischen Carbonsäuren, Carbonsäureestern und Carbonsäureamiden durch die Umsetzung von aromatischen oder heteroaromatischen Halogeniden der allgemeinen Formel I:

R(-X)ₙ (I),

worin der Index und die Variablen die folgende Bedeutung haben:
- n: ganze Zahl von 1 bis 6,
- R: substituierter oder unsubstituierter, aromatischer oder heteroaromatischer Rest und
- X: Chlor-, Brom- oder lodatom;
mit Kohlenmonoxid und Wasser, Ammoniak, Alkoholen oder Aminen in der Gegenwart von Basen und null- oder zweiwertigen Palladiumverbindungen und zweizähnigen Diphosphanen oder Komplexen von null- oder zweiwertigem Palladium mit zweizähnigen Diphosphanen, bei dem man zweizähnige Diphosphane der allgemeinen Formel II verwendet:

(R¹-)(R²-)P-Y-P(-R³)(-R⁴) (II),

worin die Variablen die folgende Bedeutung haben:
- R¹ bis R⁴: unabhängig voneinander gleich oder verschieden, unsubstituierte Arylreste oder unsubstituierte oder substituierte Cycloalkylreste; und
- Y: Kohlenwasserstoffgruppe mit insgesamt 2 bis 20 Kohlenstoffatomen, wobei mindestens eines der Kohlenstoffatome nur ein oder kein Wasserstoffatom als Substituent trägt;
ausgenommen die Umsetzung von 4-Brom-3-difluormethyl-1-methylpyrazol mit 2-(3,4,5-Trifluorphenl)anilin und Kohlenmonoxid zu N-[2-(3,4,5-Trifluorphenyl)phenyl]-3-difluormethyl-1-methylpyrazol-4-carbonsäureamid
(a) in N-Methylpyrrolidon in der Gegenwart von Pd(C₆H₅CN)₂Cl₂, 2,2-Dimethyl-1,3-bis(diphenylphosphanyl)propan und Kaliumcarbonat oder
(b) in Acetonitril in der Gegenwart von Pd(C₆H₅CN)₂Cl₂, 3,3-Bis(diphenylphosphanylmethylen)heptan, Triethylamin und Kaliumcarbonat.

### Ausführungsform 2:

Verfahren nach Ausführungsform 1, dadurch gekennzeichnet, dass die Reste R¹ bis R⁴ aus der Gruppe, bestehend aus unsubstituierten Arylresten mit 6 bis 20 Kohlenstoffatomen im Ring oder in den Ringen, sowie substituierten und unsubstituierten Cycloalkylresten mit 5 bis 16 Kohlenstoffatomen im Ring oder in den Ringen, ausgewählt werden.

### Ausführungsform 3.:

Verfahren nach Ausführungsform eins oder 2, dadurch gekennzeichnet, dass in der allgemeinen Formel II die Reste R¹ bis R⁴ gleich sind.

### Ausführungsform 4:

Verfahren nach einer der Ausführungsformen 1 bis 3, dadurch gekennzeichnet, dass die Reste R¹ bis R⁴ aus der Gruppe, bestehend aus unsubstituiertem Phenyl und Naphthyl, sowie unsubstituiertem und substituiertem Cyclopentyl und Cyclohexyl, ausgewählt werden.

### Ausführungsform 6:

Verfahren nach einer der Ausführungsformen 1 bis 4, dadurch gekennzeichnet, dass die Reste R¹ bis R⁴ unsubstituierte Phenyl- oder Cyclohexylreste sind.

### Ausführungsform 7:

Verfahren nach Ausführungsform 1 bis 4,6 dadurch gekennzeichnet, dass die Kohlenwasserstoffgruppe Y insgesamt 3 bis 15 Kohlenstoffatome enthält.

### Ausführungsform 8:

Verfahren nach einer der Ausführungsformen 1 bis 4,6,7 dadurch gekennzeichnet, dass die Kohlenwasserstoffgruppe Y ausschließlich aus Kohlenwasserstoffatomen und Wasserstoffatomen besteht.

### Ausführungsform 9:

Verfahren nach einer der Ausführungsformen 1 bis 4,6 bis 8 dadurch gekennzeichnet, dass die Kohlenwasserstoffgruppe Y die allgemeine Formel III aufweist:

-C(-R⁷)₂-C(-R⁵)(-R⁶)-C(R⁷)₂- (III).

### Ausführungsform 10:

Verfahren nach Ausführungsform 9 dadurch gekennzeichnet, dass die Reste R⁵ und R⁶ aus der Gruppe, bestehend aus
- linearen oder verzweigten Alkylresten mit einem Kohlenstoffatom oder 2 bis 12 Kohlenstoffatomen;
- substituierten und unsubstituierten Cycloalkylresten mit 5 bis 16 Kohlenstoffatomen im Ring oder in den Ringen;
- substituierten und unsubstituierten Arylresten mit 6 bis 20 Kohlenstoffatomen im Ring oder in den Ringen,
- substituierten und unsubstituierten x-Cycloalkylalkan-1-yl-Resten mit 5 bis 16 Kohlenstoffatomen im Cycloalkylrest oder substituierten und unsubstituierten x-Arylalkan-1-yl-Resten mit 6 bis 20 Kohlenstoffatomen im Arylrest sowie jeweils einem Kohlenstoffatom oder 2 bis 6 Kohlenstoffatomen im 1,x-Alkylen-Rest, wobei x = ganze Zahl von 1 bis 6; oder
- substituierten und unsubstituierten y-Arylcycloalkan-1-yl-Resten mit 6 bis 20 Kohlenstoffatomen im Arylrest und 5 bis 16 Kohlenstoffatomen im 1,y-Cycloalkylen-Rest, wobei y = ganze Zahl von 1 bis 12;
ausgewählt werden.

### Ausführungsform 11:

Verfahren nach Ausführungsform 9 oder 10, dadurch gekennzeichnet, dass in der allgemeinen Formel III keiner der Reste R⁵ und R⁶ ein Wasserstoffatom ist.

### Ausführungsform 12:

Verfahren nach einer der Ausführungsformen 9 bis 11, dadurch gekennzeichnet, dass in der allgemeinen Formel III die Reste R⁵ und R⁶ gleich sind.

### Ausführungsform 13:

Verfahren nach 11 oder 12, dadurch gekennzeichnet, dass die Reste R⁵ und R⁶ cyclisch miteinander verknüpft sind.

### Ausführungsform 14:

Verfahren nach einer der Ausführungsformen 9 bis 13, dadurch gekennzeichnet, dass die Reste R⁵ und R⁶ aus der Gruppe, bestehend aus Methyl, Ethyl, Propyl, n-Butyl, n-Pentyl, n-Hexyl, Phenyl, Benzyl, Cyclopentyl und Cyclohexyl, ausgewählt werden.

### Ausführungsform 15:

Verfahren nach einer der Ausführungsformen 9 bis 14, dadurch gekennzeichnet, dass die Reste R⁷ aus der Gruppe, bestehend aus Wasserstoffatomen, Fluoratomen, Chloratomen, Bromatomen, Nitrilgruppen, Nitrogruppen und Resten R⁵ und R⁶, ausgewählt werden.

### Ausführungsform 16:

Verfahren nach Ausführungsform 15, dadurch gekennzeichnet, dass die Reste R⁷ Wasserstoffatome sind

### Ausführungsform 17:

Verfahren nach einer der Ausführungsformen 14 bis 16, dadurch gekennzeichnet, dass die zweizähnigen Diphosphane der allgemeinen Formel II aus der Gruppe, bestehend aus
- 2-Methyl-, 2-Ethyl-, 2-Propyl-, 2-n-Butyl-, 2-n-Pentyl-, 2-n-Hexyl-, 2-Cyclohexyl-und 2-Phenyl-1,3-bis(diphenylphosphanyl)propan und -1,3-bis(dicyclohexylphosphanyl)propan,
- 2,2-Dimethyl-, 2,2-Diethyl-, 2,2-Dipropyl-, 2,2-Di-n-butyl-, 2,2-Di-n-pentyl-, 2,2-Di-n-hexyl-, 2,2-Dicyclohexyl-, 2,2-Diphenyl-1,3-bis(diphenylphosphanyl)propan und - 1,3-bis(dicyclohexylphosphanyl)propan,
- 2-Methyl-2-ethyl-, -2-propyl-, -2-n-butyl-, -2-n-pentyl-, -2-n-hexyl-, -2-cyclohexyl-und -2-phenyl-1,3-bis(diphenylphosphanyl)propan und -1,3-bis(dicyclohexylphosphanyl)propan,
- 2-Ethyl-2-propyl-, -2-n-butyl-, -2-n-pentyl-, -2-n-hexyl-, -2-cyclohexyl- und -2-phenyl-1,3-bis(diphenylphosphanyl)propan und -1,3-bis(dicyclohexylphosphanyl)propan,
- 2-Propyl-2-n-butyl-, -2-n-pentyl-, -2-n-hexyl-, -2-cyclohexyl-1,3- und -2-phenyl-1,3-bis(diphenylphosphanyl)propan und -1,3-bis(dicyclohexylphosphanyl)propan,
- 2-n-Butyl-2-n-pentyl-, -2-n-hexyl-, -2-cyclohexyl-1,3- und -2-phenyl-1,3-bis(diphenylphosphanyl)propan und -1,3-bis(dicyclohexylphosphanyl)propan,
- 2-n-Pentyl-2-n-hexyl-, -2-cyclohexyl-1,3- und -2-phenyl-1,3-bis(diphenylphosphanyl)propan und -1,3-bis(dicyclohexylphosphanyl)propan,
- 2-n-Hexyl-2-cyclohexyl- und -2-phenyl-1,3-bis(diphenylphosphanyl)propan und - 1,3-bis(dicyclohexylphosphanyl)propan und
- 2-Cyclohexyl-2-phenyl-1,3-bis(diphenylphosphanyl)propan und -1,3-bis(dicyclohexylphosphanyl)propan,
ausgewählt werden.

### Ausführungsform 18:

Verfahren nach Ausführungsform 17, dadurch gekennzeichnet, dass die zweizähnigen Diphosphane der allgemeinen Formel II aus der Gruppe, bestehend aus 2-Ethyl-2-butyl-und 2,2-Dimethyl-1,3-bis(diphenylphosphanyl)propan, ausgewählt werden.

### Ausführungsform 19.:

Verfahren nach einer der Ausführungsformen 1 bis 18, dadurch gekennzeichnet, dass sich die aromatischen Reste R der allgemeinen Formel I von Benzol und polycyclischen aromatischen Kohlenwasserstoffen und die heteroaromatischen Reste R von monocyclischen und polycyclischen aromatischen Heterocyclen ableiten.

### Ausführungsform 20:

Verfahren nach Ausführungsform 19, dadurch gekennzeichnet, dass die polycyclischen aromatischen Kohlenwasserstoffe aus der Gruppe, bestehend aus
- Kohlenwasserstoffen, worin mindestens zwei Benzolkerne, mindestens zwei kondensierte polycyclische aromatischen Kohlenwasserstoffe oder mindestens ein Benzolkern und mindestens ein kondensierter polycyclischer aromatischer Kohlenwasserstoff über mindestens eine Kohlenstoff-Kohlenstoff-Einfachbindung miteinander verknüpft sind, und
- kondensierten polycyclischen aromatischen Kohlenwasserstoffen,
ausgewählt werden.

### Ausführungsform 21:

Verfahren nach Ausführungsform 19, dadurch gekennzeichnet, dass die aromatischen Heterocyclen mindestens ein Heteroatom, ausgewählt aus der Gruppe, bestehend aus Stickstoffatom, Sauerstoffatom und Schwefelatom, enthalten.

### Ausführungsform 22:

Verfahren nach einer der Ausführungsformen 1 bis 4, 6 bis 21 dadurch gekennzeichnet, dass der Alkohol aus der Gruppe, bestehend aus aliphatischen, cycloaliphatischen, aromatischen und heteroaromatischen Alkoholen mit 1 bis 4 Hydroxylgruppen im Molekül, ausgewählt wird.

### Ausführungsform 23:

Verfahren nach einer der Ausführungsformen 1 bis 4, 6 bis 22 dadurch gekennzeichnet, dass das Amin aus der Gruppe, bestehend aus aliphatischen, cycloaliphatischen, cyclischen, aromatischen und heteroaromatischen, primären und sekundären Aminen mit 1 bis 4 Aminogruppen im Molekül, ausgewählt wird.

### Ausführungsform 24:

Verfahren nach Ausführungsform 22 oder 23, dadurch gekennzeichnet, dass der jeweilige Grundkörper der Alkohole und/oder der Amine mit mindestens einem inerten Substituenten substituiert ist.

### Ausführungsform 25:

Verfahren nach einer der Ausführungsformen 1 bis 4, 6 bis 24 dadurch gekennzeichnet, dass die nullwertige Palladiumverbindung aus der Gruppe, bestehend aus metallischem Palladium und Palladium(0)-organylen und die zweiwertige Palladiumverbindung aus der Gruppe, bestehend aus Palladium(II)-organylen und Palladium(II)-salzen, ausgewählt wird.

### Ausführungsform 26:

Verfahren nach Ausführungsform 25, dadurch gekennzeichnet, dass das metallische Palladium geträgert ist.

### Ausführungsform 27:

Verfahren nach Ausführungsform 25, dadurch gekennzeichnet, dass das Palladium(0)-organyl aus der Gruppe, bestehend aus Tris(η²-alken)-palladium(0), Bis(carben)-palladium(0), Palladium(0)-Phosphankomplexen und gemischten Palladium(0)-(η²-Alken)-Phosphankomplexen, das Palladium(II)-organyl aus der Gruppe, bestehend aus Palladium(II)-Chelatkomplexen, Donoraddukten mit einwertigen Liganden und pi-gebundenen Liganden und Komplexen mit einwertigen Liganden und Halogeniden, und die Palladium(II)-salze aus der Gruppe, bestehend aus Palladium(II)-halogeniden und - carboxylaten, ausgewählt wird.

### Ausführungsform 28:

Verfahren nach einer der Ausführungsformen 1 bis 4, 6 bis 27 dadurch gekennzeichnet, dass die Base aus der Gruppe, bestehend aus Alkalimetallsalzen, Alkoholaten, überschüssigen Aminen, wie in Ausführungsform 23 definiert, und tertiären Aminen, ausgewählt wird.

### Ausführungsform 29:

Verfahren nach einer der Ausführungsformen 1 bis 4, 6 bis 26 dadurch gekennzeichnet, dass die aromatischen oder heteroaromatischen Halogenide der allgemeinen Formel I mit Wasser, Alkoholen oder Aminen in einem Molverhältnis von Halogenid I : Wasser, Alkohol oder Amin von 0,5 : 1 bis 2 : 1, bezogen auf das jeweilige nucleophile Sauerstoffatom oder Stickstoffatom, umgesetzt werden.

### Ausführungsform 30.: 4, 6 bis 29

Verfahren nach einer der Ausführungsformen 1 bis 4, 6 bis 29 dadurch gekennzeichnet, dass die nullwertige oder zweiwertige Palladiumverbindung in einer Menge von 0,001 bis 5 Mol-%, bezogen auf das Halogenid I, eingesetzt wird.

### Ausführungsform 31:

Verfahren nach einer der Ausführungsformen 1 bis 4, 6 bis 28 dadurch gekennzeichnet, dass die Base in einem Äquivalentverhältnis von Halogenatom im Halogenid I zu Base = 1 : 1 bis 1 : 4 verwendet wird.

### Ausführungsform 32:

Verfahren nach einer der Ausführungsformen 1 bis 4, 6 bis 31 dadurch gekennzeichnet, dass die Umsetzung in einem aromatischen oder einem aprotisch polaren organischen Lösemittel durchgeführt wird.

### Ausführungsform 33:

Verfahren nach Ausführungsform 32, dadurch gekennzeichnet, dass das organische Lösemittel aus der Gruppe, bestehend aus überschüssigen aromatischen oder heteroaromatischen Halogeniden der allgemeinen Formel I, Toluol, Xylolen, Amiden, Ethern, Sulfonen und Nitrilen, ausgewählt wird.

### Ausführungsform 34:

Verfahren nach Ausführungsform 32 oder 33, dadurch gekennzeichnet, dass das organische Lösemittel bei der Umsetzung der Halogenide I mit Alkoholen oder Aminen im Wesentlichen oder völlig wasserfrei ist.

### Ausführungsform 35:

Verfahren nach einer der Ausführungsformen 1 bis 4, 6 bis 34 dadurch gekennzeichnet, dass zusätzlich zu den Diphosphanen der allgemeinen Formel II oder ihren Komplexen mit null-oder zweiwertigem Palladium noch mindestens ein organisches Monophosphan verwendet wird

### Ausführungsform 36:

Verfahren nach einer der Ausführungsformen 1 bis 4, 6 bis 35 dadurch gekennzeichnet, dass die Umsetzung bei einer Temperatur zwischen 90 und 200°C durchgeführt wird.

### Ausführungsform 37:

Verfahren nach einer der Ausführungsformen 1 bis 4, 6 bis 36 dadurch gekennzeichnet, dass die Umsetzung bei einem Kohlenmonoxid-Partialdruck von 0,9 bis 100 bar (90 bis 10.000 kPa) durchgeführt wird.

### Beispiele und Vergleichsversuche

### Herstellbeispiel

### Herstellung von Pd[2,2-dimethyl-1,3-bis-(diphenylphosphanyl)propan]₂Cl₂, Pd(Pepstar)₂Cl₂

Die Darstellung von Pd(Pepstar)₂Cl₂ erfolgte in Analogie zur M. R. Mason, J. G. Verkade, Organometallics, 1992, 11, 2212-2220.

Pd(PhCN)₂Cl₂ (0.31 mmol) wurde in einem Schlenk-Kolben vorgelegt. Sukzessiv wurden insgesamt 50 ml Toluol zum Lösen zugegeben. Pepstar (0.33 mmol) wurde in 5 ml Toluol gelöst und zur orangefarbenen Pd-Lösung gegeben. Dabei fiel ein blassgelber Feststoff aus, während sich die Lösung entfärbte.

Nach 1 h Nachrühren filtrierte man den Feststoff ab und wusch dreimal mit je 3 ml Hexan. Der Komplex wurde im Vakuum bis zur Gewichtskonstanz getrocknet. PdCl₂(Pepstar)₂ wurde in quantitativer Ausbeute (191.5 mg) erhalten.

### Beispiele 1 bis 5-4

### Carboxymethylierung von Chloracetophenon zu 4-Acetylbenzoesäuremethylester

### Reaktionsgleichung:

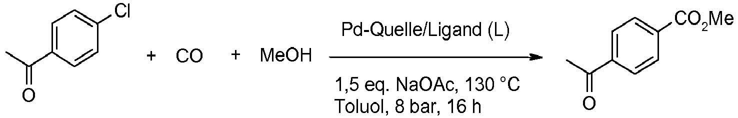

### Beispiel 1:

### Methoxycarbonylierung mit 2,2-Dimethyl-1,3-bis(diphenylphosphanyl)propan (Pepstar) als Ligand (Methode A)

Der Katalysator Pd(OAc)₂ (Ac = acetyl; 0.3 mol-%) und der Ligand 2,2-Dimethyl-1,3-bis(diphenylphosphino)propan (Pepstar; 1.1 mol-%) wurden in einer Glovebox unter Schutzgas eingewogen und in 5 ml entgastem Methanol gelöst. Anschließend wurden 20 ml entgastes Toluol, das Substrat 4-Acetylchlorbenzol (10 mmol) und Natriumacetat (15 mmol) zu dem Ansatz gegeben.

Der Reaktionsansatz wurde in einen, mit Argon gespülten Autoklaven überführt, und der Autoklav wurde dreimal mit Kohlenmonoxid gespült. Anschließend wurde die Reaktionslösung auf 130°C erhitzt. Anschließend wurden 6 bar CO aufgepresst. Die Reaktion wurde 16 h bei 8 bar und 130°C gefahren. Der Autoklav wurde abgekühlt und geöffnet. Die gaschromatographische Analyse ergab nach 16 h einen quantitativen Umsatz bei einer Selektivität von 85%.

### Analyse:

DB-1, 5 min 60°C; 10°/min bis 260°C

| Retentionszeit [min] | Verbindung |
|---|---|
| 14.67 | 4-Chloracetophenon |
| 18.28 | Produkt |

### Beispiel 2:

### Methoxycarbonylierung mit 2,2-Dimethyl-1,3-bis(diphenylphosphanyl)propan (Pepstar) als Ligand (modifizierte Methode A)

Der Katalysator Pd(OAc)₂ (0.1 mol-%) und der Ligand 2,2-Dimethyl-1,3-bis(diphenylphosphino)propan (Pepstar, 1.1 mol-%) wurden in einer Glovebox unter Schutzgas eingewogen und in 5 ml entgastem Methanol gelöst. Anschließend wurden 20 ml entgastes Toluol, das Substrat 4-Acetylchlorbenzol (10 mmol) und Natriumacetat (15 mmol) zu dem Ansatz gegeben.

Der Reaktionsansatz wurde in einen, mit Argon gespülten Autoklaven überführt, und der Autoklav wurde dreimal mit Kohlenmonoxid gespült. Anschließend wurde die Reaktionslösung auf 130°C erhitzt. Anschließend wurden 6 bar CO aufgepresst. Die Reaktion wird 16 h bei 8 bar und 130°C gefahren. Der Autoklav wurde abgekühlt und geöffnet. Die gaschromatographische Analyse ergab nach 16 h einen Umsatz von 99% bei einer Selektivität von 82%.

### Beispiel 3:

### Methoxycarbonylierung mit Pd(Pepstar)₂Cl₂ (Methode B)

Der Katalysator Pd(Pepstar)₂Cl₂ (0.1 mol-%; vgl. das Herstellbeispiel) und der Ligand 2,2-Dimethyl-1,3-bis(diphenylphosphino)propan (Pepstar, 0.3 mol-%) wurden in einer Glovebox unter Schutzgas eingewogen und in 5 ml entgastem Methanol gelöst. Anschließend wurden 20 ml entgastes Toluol, das Substrat 4-Acetylchlorbenzol (10 mmol) und NaOAc (15 mmol) zu dem Ansatz gegeben.

Der Reaktionsansatz wurde in einen, mit Argon gespülten Autoklaven überführt, und der Autoklav wurde dreimal mit Kohlenmonoxid gespült. Anschließend wurde die Reaktionslösung auf 130 °C erhitzt. Anschließend wurden 6 bar CO aufgepresst. Die Reaktion wurde 24 h bei 8 bar und 130°C gefahren. Der Autoklav wurde abgekühlt und geöffnet. Die gaschromatographische Analyse ergab nach 24 h einen Umsatz von 99% bei einer Selektivität von 82.6%.

### Beispiel 4:

### Methoxycarbonylierung mit Pd(Pepstar)₂Cl₂ (Methode C)

Der Katalysator Pd(Pepstar)₂Cl₂ (0.1 mol-%) wurde in einer Glovebox unter Schutzgas eingewogen und in 5 ml entgastem Methanol gelöst. Anschließend wurden 20 ml entgastes Toluol, das Substrat 4-Acetylchlorbenzol (10 mmol) und NaOAc (15 mmol) zu dem Ansatz gegeben.

Der Reaktionsansatz wurde in einen, mit Argon gespülten Autoklaven überführt, und der Autoklav wurde dreimal mit Kohlenmonoxid gespült. Anschließend wurde die Reaktionslösung auf 130°C erhitzt. Anschließend wurden 6 bar CO aufgepresst. Die Reaktion wurde 16 h bei 8 bar und 130°C gefahren. Der Autoklav wurde abgekühlt und geöffnet. Die gaschromatographische Analyse ergab nach 16 h einen Umsatz von 85% bei einer Selektivität von 81.7%.

### Beispiele 5-1 bis 5-4:

### Methoxycarbonylierung mit Pd(Pepstar)₂Cl₂ und Triphenylphosphan (Methode D)

### Allgemeine Versuchsvorschrift:

Der Katalysator Pd(Pepstar)₂Cl₂ (0.1 mol-%) und der Ligand Triphenylphosphan wurden in einer Glovebox unter Schutzgas eingewogen und in 5 ml entgastem Methanol gelöst. Anschließend wurden 20 ml entgastes Toluol, das Substrat 4-Acetylchlorbenzol (10 mmol) und NaOAc (15 mmol) zu dem Ansatz gegeben.

Der Reaktionsansatz wurde in einen, mit Argon gespülten Autoklaven überführt und der Autoklav wurde dreimal mit Kohlenmonoxid gespült. Anschließend wurde und die Reaktionslösung auf 130°C erhitzt. Anschließend wurden 6 bar CO aufgepresst. Die Reaktion wurde 16 h bei 8 bar und 130°C gefahren. Der Autoklav wurde abgekühlt und geöffnet. Nach 16 h beziehungsweise 24 h wurden die Umsätze und Selektivitäten mithilfe der Gaschromatographie ermittelt. Die nachstehende Übersicht Nr. 1 gibt einen Überblick über die Molverhältnisse von Pd(Pepstar)₂Cl₂ : Triphenylphosphin, die Umsätze und die Selektivitäten.

**Übersicht Nr. 1:**

| Nr. 5- | Pd(Pepstar)₂Cl₂: Triphenylphosphin | Umsatz [%] | Selektivität [%] |
|---|---|---|---|
| 1 | 1:3 | 74.5 | 80.2 |
| 2* | 1:5 | 92.4 | 83.7 |
| 3* | 1:7.5 | 84.0 | 100.0 |
| 4* | 1:10 | 82.3 | 100.0 |

| | | | |
|---|---|---|---|
| * Die Reaktionszeit betrug bei diesen Versuchen 24 h. | | | |

### Beispiele 6-1 bis 6-3 und 7

### Amidocarbonylierung von Chloracetophenon mit Anilin zu 4-Acetyl-N-phenyl-benzamid

### Reaktionsgleichung

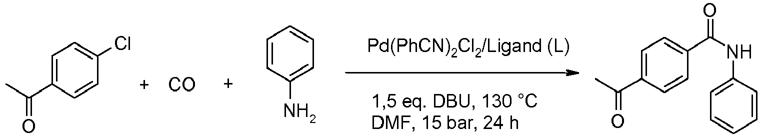

### Beispiel 6-1:

### Amidocarbonylierung mit 2,2-Dimethyl-1,3-bis(diphenylphosphanyl)propan (Pepstar) als Ligand

### Allgemeine Versuchsvorschrift:

Der Katalysator Pd(PhCN)₂Cl₂ (0.5 mol-%) und der Ligand 2,2-Dimethyl-1,3-bis(diphenyl-phosphanyl)propan (Pepstar, 1.5 mol-%) wurden in einer Glovebox unter Schutzgas eingewogen und in 5 ml entgastem DMF gelöst. Anschließend wurden 20 ml entgastes DMF, das Substrat 4-Acetylchlorbenzol (10 mmol), Anilin (15 mmol) und Base (15 mmol) zu dem Ansatz gegeben.

Der Reaktionsansatz wurde in einen, mit Argon gespülten Autoklaven überführt, und der Autoklav wurde dreimal mit Kohlenmonoxid gespült. Anschließend wurde die Reaktionslösung auf 130°C erhitzt. Anschließend wurden 15 bar CO aufgepresst. Die Reaktion wurde 24 h bei 15 bar und 130°C beziehungsweise 150°C gefahren. Der Autoklav wurde abgekühlt und geöffnet. Eine gaschromatographische Analyse des Reaktionsaustrags wurde gemäß der folgenden Übersicht Nr. 2 durchgeführt.

### Analyse:

DB-1, 5 min 60°C; 10°/min bis 260°C

**Übersicht Nr. 2:**

| Retentionszeit [min] | Verbindung |
|---|---|
| 9.40 | Anilin |
| 14.72 | 4-Chloracetophenon |
| 29.54 | Produkt |

Die nachstehende Übersicht Nr. 3 zeigt die bei den Beispielen 6-1 bis 6-3 mit verschiedenen Basen erhaltenen Umsätze und Selektivitäten.

**Übersicht Nr. 3:**

| Nr. 6- | Basen | Umsatz [%] | Selektivität [%] |
|---|---|---|---|
| 1 | DBU^{a)} | 99.1 | 70.1 |
| 2* | NEt₃^{b)} | 89.1 | 29.4 |
| 3* | K₂CO₃ | 96.7 | 78.0 |

| | | | |
|---|---|---|---|
| *Die Reaktionstemperatur betrug bei diesen Versuchen 150°C. a) 1,8-Diazabicyclo[5.4.0]undec-7-en b) Triethylamin | | | |

### Beispiel 7:

### Amidocarbonylierung mit 2-Butyl-2-ethyl-1,3-bis(diphenylphosphanyl)propan (Bustar) als Ligand

Der Katalysator Pd(PhCN)₂Cl₂ (0.5 mol-%) und der Ligand 2-Butyl-2-ethyl-1,3-bis(diphenylphosphanyl)propan (Bustar; 1.5 mol-%) wurden in einer Glovebox unter Schutzgas eingewogen und in 5 ml entgastem DMF gelöst. Anschließend wurden 20 ml entgastes DMF, das Substrat 4-Acetylchlorbenzol (10 mmol), Anilin (15 mmol) und DBU (15 mmol) zu dem Ansatz gegeben.

Der Reaktionsansatz wurde in einen, mit Argon gespülten Autoklaven überführt, und der Autoklav wurde dreimal mit Kohlenmonoxid gespült. Anschließend wurde die Reaktionslösung auf 130°C erhitzt. Anschließend wurden 15 bar CO aufgepresst. Die Reaktion wurde 16 h bei 15 bar und 130°C gefahren. Der Autoklav wurde abgekühlt und geöffnet. Die gaschromatographische Analyse ergab nach 24 h einen Umsatz von 97.7% und eine Selektivität von 91.4%.

### Beispiele 8 und 9 und Vergleichsversuche V1 und V2

### Carboxylierung von 4-Bromtoluol zu 4-Methylbenzoesäure

### Reaktionsgleichung:

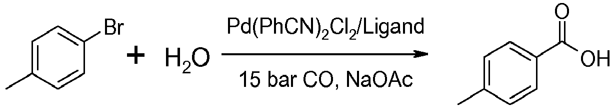

### Allgemeine Versuchsvorschrift:

In einen Reaktorblock, bestehend aus vier Parallelreaktoren, wurde NaOAc (4.1 mmol) vorgelegt. Pd(OAc)₂ (0.028 mmol, 1 mol-%) und der entsprechende Ligand (0.084 mmol, 3 mol-%) wurden in DMF (10 ml) zugegeben. Das resultierende Reaktionsgemisch wurde während 30 min bei Raumtemperatur geschüttelt. Anschließend wurden 4-Bromtoluol (2.8 mmol) und Wasser (1 ml) zudosiert. Nach 10 min bei 15 bar CO und Raumtemperatur wurde das Reaktionsgemisch auf 140°C erhitzt. Nach 16 h wurden die Reaktoren abgekühlt und entspannt. Der Reaktoraustrag wurde mittels Gaschromatographie analysiert (vgl. die Übersicht Nr. 4).

### Analyse:

DB-1, 5 min. 60°C; 10°/min bis 260°C

**Übersicht Nr. 4:**

| Retentionszeit [min] | Verbindung |
|---|---|
| 11.22 | 4-Bromtoluol |
| 15.51 | 4-Methylbenzoesäure |

Die bei den Beispielen 8 und 9 und den Vergleichsversuchen V1 und V2 eingesetzten Mengenverhältnisse sowie die nach 16 h erhaltenen Umsätze und Selektivitäten sind in der nachstehenden Übersicht Nr. 5 dargestellt.

**Übersicht Nr. 5:**

| | Beispiel 8 Reaktor 1 | Beispiel 9 Reaktor 2 | Vergleich V1 Reacktor 3 | Vergleich V2 Reaktor 4 |
|---|---|---|---|---|
| Ligand | Pepstar | Bustar | DPP^{a)} | DPPF^{b)} |
| Edukt [%] | 0.39 | 0.50 | 0.58 | 0.93 |
| Produkt [%] | 5.91 | 6.07 | 3.12 | 2.21 |
| Umsatz [%] | 97.2 | 96.3 | 94.9 | 88.4 |
| Selektivität [%] | 44.2 | 47.1 | 28.7 | 31.2 |

| | | | | |
|---|---|---|---|---|
| a) 1,3-Bis(diphenylphosphanyl)propan b) 1,1 '-Bis(diphenylphosphanyl)ferrocen | | | | |

Der Vergleich der Umsätze und der Selektivitäten untermauerte, dass Pepstar und Bustar den anderen Liganden eindeutig überlegen war

## Patentansprüche

1. Verfahren zur Herstellung von aromatischen und heteroaromatischen Carbonsäuren, Carbonsäureestern und Carbonsäureamiden durch die Umsetzung von aromatischen oder heteroaromatischen Halogeniden der allgemeinen Formel I:
R(-X)ₙ (I),
worin der Index und die Variablen die folgende Bedeutung haben:
n ganze Zahl von 1 bis 6,
R substituierter oder unsubstituierter, aromatischer oder heteroaromatischer Rest und
X Chlor-, Brom- oder lodatom;
mit Kohlenmonoxid und Wasser, Ammoniak, Alkoholen oder Aminen in der Gegenwart von Basen und null- oder zweiwertigen Palladiumverbindungen und zweizähnigen Diphosphanen oder Komplexen von null- oder zweiwertigem Palladium mit zweizähnigen Diphosphanen, bei dem man zweizähnige Diphosphane der allgemeinen Formel II verwendet:
(R¹-)(R²-)P-Y-P(-R³)(-R⁴) (II),
worin die Variablen die folgende Bedeutung haben:
R¹ bis R⁴ unabhängig voneinander gleich oder verschieden, unsubstituierte Arylreste oder unsubstituierte oder substituierte Cycloalkylreste; und
Y Kohlenwasserstoffgruppe mit insgesamt 2 bis 20 Kohlenstoffatomen, wobei mindestens eines der Kohlenstoffatome nur ein oder kein Wasserstoffatom als Substituent trägt;
ausgenommen die Umsetzung von 4-Brom-3-difluormethyl-1-methylpyrazol mit 2-(3,4,5-Trifluorphenl)anilin und Kohlenmonoxid zu N-[2-(3,4,5-Trifluorphenyl)phenyl]-3-difluormethyl-1-methylpyrazol-4-carbonsäureamid
(a) in N-Methylpyrrolidon in der Gegenwart von Pd(C₆H₅CN)₂Cl₂, 2,2-Dimethyl-1,3-bis(diphenylphosphanyl)propan und Kaliumcarbonat oder
(b) in Acetonitril in der Gegenwart von Pd(C₆H₅CN)₂Cl₂, 3,3-Bis(diphenylphosphanylmethylen)heptan, Triethylamin und Kaliumcarbonat.

2. Verfahren nach Anspruch 1, **dadurch gekennzeichnet, dass** die Kohlenwasserstoffgruppe Y ausschließlich aus Kohlenwasserstoffatomen und Wasserstoffatomen besteht.

3. Verfahren nach Anspruch 1 oder 2, **dadurch gekennzeichnet, dass** die Kohlenwasserstoffgruppe Y die allgemeine Formel III aufweist:
-COR⁷)₂-COR⁵)OR⁶OC(R⁷)₂- (III);
worin die Variablen R⁵ und R⁶ unabhängig voneinander gleich oder verschieden sind und für Wasserstoffatome, substituierte oder unsubstituierte, lineare oder verzweigte Alkylreste, substituierte oder unsubstituierte Cycloalkylreste oder Arylreste oder Reste, die mindestens zwei dieser Reste enthalten oder hieraus bestehen, stehen, wobei nur einer der Reste R⁵ und R⁶ ein Wasserstoffatom ist; und worin die Variable R⁷ für Wasserstoffatom, Fluoratom, Chloratom, Bromatom, Nitrilgruppe, Nitrogruppe oder Rest R⁵ oder R⁶ steht.

4. Verfahren nach Anspruch 3, **dadurch gekennzeichnet, dass** in der allgemeinen Formel III keiner der Reste R⁵ und R⁶ ein Wasserstoffatom ist.

5. Verfahren nach Anspruch 2 oder 4, **dadurch gekennzeichnet, dass** in der allgemeinen Formel III die Reste R⁵ und R⁶ gleich sind.

6. Verfahren nach Anspruch 4 oder 5, **dadurch gekennzeichnet, dass** die Reste R⁵ und R⁶ cyclisch miteinander verknüpft sind.

7. Verfahren nach einem der Ansprüche 1 bis 6, **dadurch gekennzeichnet, dass** sich die aromatischen Reste R der allgemeinen Formel I von Benzol und polycyclischen aromatischen Kohlenwasserstoffen und die heteroaromatischen Reste R von monocyclischen und polycyclischen aromatischen Heterocyclen ableiten.

8. Verfahren nach einem der Ansprüche 1 bis 7, **dadurch gekennzeichnet, dass** der Alkohol aus der Gruppe, bestehend aus aliphatischen, cycloaliphatischen, aromatischen und heteroaromatischen Alkoholen mit 1 bis 4 Hydroxylgruppen im Molekül, ausgewählt wird.

9. Verfahren nach einem der Ansprüche 1 bis 7, **dadurch gekennzeichnet, dass** das Amin aus der Gruppe, bestehend aus aliphatischen, cycloaliphatischen, cyclischen, aromatischen und heteroaromatischen, primären und sekundären Aminen mit 1 bis 4 Aminogruppen im Molekül, ausgewählt wird.

10. Verfahren nach einem der Ansprüche 1 bis 9, **dadurch gekennzeichnet, dass** die nullwertige Palladiumverbindung aus der Gruppe, bestehend aus metallischem Palladium und Palladium(0)-organylen und die zweiwertige Palladiumverbindung aus der Gruppe, bestehend aus Palladium(II)-organylen und Palladium(II)-salzen, ausgewählt wird.

11. Verfahren nach einem der Ansprüche 1 bis 10, **dadurch gekennzeichnet, dass** die Base aus der Gruppe, bestehend aus Alkalimetallsalzen, Alkoholaten, überschüssigen Aminen, wie in Anspruch 9 definiert, und tertiären Aminen, ausgewählt wird.

12. Verfahren nach einem der Ansprüche 1 bis 11, **dadurch gekennzeichnet, dass** die aromatischen oder heteroaromatischen Halogenide der allgemeinen Formel I mit Wasser, Alkoholen oder Aminen in einem Molverhältnis von Halogenid I : Wasser, Alkohol oder Amin von 0,5 : 1 bis 2 : 1, bezogen auf das jeweilige nucleophile Sauerstoffatom oder Stickstoffatom, umgesetzt werden.

13. Verfahren nach einem der Ansprüche 1 bis 12, **dadurch gekennzeichnet, dass** die nullwertige oder zweiwertige Palladiumverbindung in einer Menge von 0,001 bis 5 Mol-%, bezogen auf das Halogenid I, eingesetzt wird.

14. Verfahren nach einem der Ansprüche 1 bis 13, **dadurch gekennzeichnet, dass** die Base in einem Äquivalentverhältnis von Halogenatom im Halogenid I zu Base = 1 : 1 bis 1 : 4 verwendet wird.

15. Verfahren nach einem der Ansprüche 1 bis 14, **dadurch gekennzeichnet, dass** die Umsetzung in einem aromatischen oder einem aprotisch polaren organischen Lösemittel durchgeführt wird.

## Claims

1. A process for the preparation of aromatic and heteroaromatic carboxylic acids, carboxylic acid esters and carboxamides by the reaction of aromatic or heteroaromatic halides of the general formula I:
R(-X)ₙ (I),
in which the index and the variables have the following meanings:
n integer from 1 to 6,
R substituted or unsubstituted and aromatic or heteroaromatic radical and
X chlorine, bromine or iodine atom;
with carbon monoxide and water, ammonia, alcohols or amines in the presence of bases and zero-valent or divalent palladium compounds and bidentate diphosphanes or complexes of zero- or divalent palladium with bidentate diphosphanes, in which use is made of bidentate diphosphanes of the general formula II:
(R¹-)(R²-)P-Y-P(-R³)(-R⁴) (II),
in which the variables have the following meanings:
R¹ to R⁴ independently of one another, identically or differently, unsubstituted aryl radicals or unsubstituted or substituted cycloalkyl radicals; and
Y hydrocarbon group with a total of 2 to 20 carbon atoms, in which at least one of the carbon atoms carries only one or no hydrogen atom as substituent;
except for the reaction of 4-bromo-3-difluoromethyl-1-methylpyrazole with 2-(3,4,5-trifluorophenyl)aniline and carbon monoxide to give N-[2-(3,4,5-trifluorophenyl)phenyl]-3-difluoromethyl-1-methylpyrazole-4-carboxamide,
(a) in N-methylpyrrolidone in the presence of Pd(C₆H₅CN)₂Cl₂, 2,2-dimethyl-1,3-bis(diphenylphosphanyl)propane and potassium carbonate or
(b) in acetonitrile in the presence of Pd(C₆H₅CN)₂Cl₂, 3,3-bis(diphenylphosphanylmethylene)heptane, triethylamine and potassium carbonate.

2. The process according to claim 1, wherein the hydrocarbon group Y consists exclusively of hydrocarbon atoms and hydrogen atoms.

3. The process according to claim 1 or 2, wherein the hydrocarbon group Y exhibits the general formula III:
-C(-R⁷)₂-C(-R⁵)(-R⁶)-C(R⁷)₂- (III);
in which the variables R⁵ and R⁶ are, independently of one another, identical or different and represent hydrogen atoms, substituted or unsubstituted and linear or branched alkyl radials, substituted or unsubstituted cycloalkyl radicals or aryl radicals or radicals comprising or consisting of at least two of the radicals, only one of these radicals R⁵ and R⁶ being a hydrogen atom; and in which the variable R⁷ represents a hydrogen atom, fluorine atom, chlorine atom, bromine atom, nitrile group, or nitro group or radical R⁵ or R⁶.

4. The process according to claim 3, wherein, in the general formula III, neither of the radicals R⁵ and R⁶ is a hydrogen atom.

5. The process according to claim 2 or 4, wherein, in the general formula III, the radicals R⁵ and R⁶ are identical.

6. The process according to claim 4 or 5, wherein the radicals R⁵ and R⁶ are linked cyclically to one another.

7. The process according to any of claims 1 to 6, wherein the aromatic radicals R of the general formula I are derived from benzene and polycyclic aromatic hydrocarbons and the heteroaromatic radicals R are derived from monocyclic and polycyclic aromatic heterocycles.

8. The process according to any of claims 1 to 7, wherein the alcohol is chosen from the group consisting of aliphatic, cycloaliphatic, aromatic and heteroaromatic alcohols with from 1 to 4 hydroxyl groups in the molecule.

9. The process according to any of claims 1 to 7, wherein the amine is chosen from the group consisting of aliphatic, cycloaliphatic, cyclic, aromatic and heteroaromatic primary and secondary amines with from 1 to 4 amino groups in the molecule.

10. The process according to any of claims 1 to 9, wherein the zero-valent palladium compound is chosen from the group consisting of metallic palladium and organopalladium(0) compounds and the divalent palladium compound is chosen from the group consisting of organopalladium(II) compounds and palladium(II) salts.

11. The process according to any of claims 1 to 10, wherein the base is chosen from the group consisting of alkali metal salts, alkoxides, excess amines, as defined in claim 9, and tertiary amines.

12. The process according to any of claims 1 to 11, wherein the aromatic or heteroaromatic halides of the general formula I are reacted with water, alcohols or amines in a molar ratio of halide I : water, alcohol or amine of 0.5 : 1 to 2 : 1, based on the respective nucleophilic oxygen atom or nitrogen atom.

13. The process according to any of claims 1 to 12, wherein the zero-valent or divalent palladium compound is used in an amount of 0.001 to 5 mol%, based on the halide I.

14. The process according to any of claims 1 to 13, wherein the base is used in an equivalent ratio of halogen atom in the halide I to base = 1 : 1 to 1 : 4.

15. The process according to any of claims 1 to 14, wherein the reaction is carried out in an aromatic solvent or a polar aprotic organic solvent.

## Revendications

1. Procédé de fabrication d'acides carboxyliques aromatiques et hétéroaromatiques, d'esters d'acides carboxyliques et d'amides d'acides carboxyliques par la mise en réaction d'halogénures aromatiques ou hétéroaromatiques de formule générale I :
R(-X)ₙ (I),
dans laquelle l'indice et les variables ont la signification suivante :
n nombre entier de 1 à 6,
R radical aromatique ou hétéroaromatique substitué ou non substitué, et
X atome de chlore, de brome ou d'iode ;
avec du monoxyde de carbone et de l'eau, de l'ammoniac, des alcools ou des amines en présence de bases et de composés de palladium zéro- ou bivalents et de diphosphanes bidentates ou de complexes de palladium zéro- ou bivalents avec des diphosphanes bidentates, selon lequel des diphosphanes bidentates de formule générale II sont utilisés :
(R¹-)(R²-)P-Y-P(-R³)(-R⁴) (II),
dans laquelle les variables ont la signification suivante :
R¹ à R⁴ indépendamment les uns des autres, radicaux aryle non substitués ou radicaux cycloalkyle non substitués ou substitués identiques ou différents ; et
Y groupe hydrocarboné contenant au total 2 à 20 atomes de carbone, au moins un des atomes de carbone ne portant qu'un seul ou pas d'atome d'hydrogène en tant que substituant ;
à l'exception de la mise en réaction de 4-bromo-3-difluorométhyl-1-méthylpyrazole avec de la 2-(3,4,5-trifluorophényl)aniline et du monoxyde de carbone en amide de l'acide N-[2-(3,4,5-trifluorophényl)phényl]-3-difluorométhyl-1-méthylpyrazole-4-carboxylique,
(a) dans de la N-méthylpyrrolidone en présence de Pd(C₆H₅CN)₂Cl₂, de 2,2-diméthyl-1,3-bis(diphénylphosphanyl)propane et de carbonate de potassium, ou
(b) dans de l'acétonitrile en présence de Pd(C₆H₅CN)₂Cl₂, de 3,3-bis(diphénylphosphanylméthylène)heptane, de triéthylamine et de carbonate de potassium.

2. Procédé selon la revendication 1, **caractérisé en ce que** le groupe hydrocarboné Y est exclusivement constitué d'atomes d'hydrocarbure et d'atomes d'hydrogène.

3. Procédé selon la revendication 1 ou 2, **caractérisé en ce que** le groupe hydrocarboné Y présente la formule générale III :
-C(-R⁷)₂-C(-R⁵)(-R⁶)-C(R⁷)₂- (III)
dans laquelle les variables R⁵ et R⁶ sont indépendamment l'une de l'autre identiques ou différentes, et représente des atomes d'hydrogène, des radicaux alkyle substitués ou non substitués, linéaires ou ramifiés, des radicaux cycloalkyle substitués ou non substitués ou des radicaux aryle, ou des radicaux qui contiennent au moins deux de ces radicaux ou en sont constitués, seul un des radicaux R⁵ et R⁶ étant un atome d'hydrogène ; et dans laquelle la variable R⁷ représente un atome d'hydrogène, un atome de fluor, un atome de chlore, un atome de brome, un groupe nitrile, un groupe nitro ou le radical R⁵ ou R⁶.

4. Procédé selon la revendication 3, **caractérisé en ce que**, dans la formule générale III, aucun des radicaux R⁵ et R⁶ n'est un atome d'hydrogène.

5. Procédé selon la revendication 2 ou 4, **caractérisé en ce que**, dans la formule générale III, les radicaux R⁵ et R⁶ sont identiques.

6. Procédé selon la revendication 4 ou 5, **caractérisé en ce que** les radicaux R⁵ et R⁶ sont reliés l'un à l'autre en un cycle.

7. Procédé selon l'une quelconque des revendications 1 à 6, **caractérisé en ce que** les radicaux aromatiques R de la formule générale I dérivent de benzène et d'hydrocarbures aromatiques polycycliques, et les radicaux hétéroaromatiques R dérivent d'hétérocycles aromatiques monocycliques et polycycliques.

8. Procédé selon l'une quelconque des revendications 1 à 7, **caractérisé en ce que** l'alcool est choisi dans le groupe constitué par les alcools aliphatiques, cycloaliphatiques, aromatiques et hétéroaromatiques, contenant 1 à 4 groupes hydroxyle par molécule.

9. Procédé selon l'une quelconque des revendications 1 à 7, **caractérisé en ce que** l'amine est choisie dans le groupe constitué par les amines primaires et secondaires aliphatiques, cycloaliphatiques, cycliques, aromatiques et hétéroaromatiques, contenant 1 à 4 groupes amino par molécule.

10. Procédé selon l'une quelconque des revendications 1 à 9, **caractérisé en ce que** le composé de palladium zéro-valent est choisi dans le groupe constitué par le palladium métallique et les organyles de palladium (0), et le composé de palladium bivalent dans le groupe constitué par les organyles de palladium (II) et les sels de palladium (II).

11. Procédé selon l'une quelconque des revendications 1 à 10, **caractérisé en ce que** la base est choisie dans le groupe constitué par les sels de métaux alcalins, les alcoolates, les amines en excès, telles que définies dans la revendication 9, et les amines tertiaires.

12. Procédé selon l'une quelconque des revendications 1 à 11, **caractérisé en ce que** les halogénures aromatiques et hétéroaromatiques de formule générale I sont mis en réaction avec de l'eau, des alcools ou des amines en un rapport molaire halogénure I:eau, alcool ou amine de 0,5:1 à 2:1, par rapport à l'atome d'oxygène ou l'atome d'azote nucléophile respectif.

13. Procédé selon l'une quelconque des revendications 1 à 12, **caractérisé en ce que** le composé de palladium zéro-valent ou bivalent est utilisé en une quantité de 0,001 à 5 % en moles, par rapport à l'halogénure I.

14. Procédé selon l'une quelconque des revendications 1 à 13, **caractérisé en ce que** la base est utilisée en un rapport équivalent entre l'atome d'halogène dans l'halogénure I et la base = 1:1 à 1:4.

15. Procédé selon l'une quelconque des revendications 1 à 14, **caractérisé en ce que** la réaction est réalisée dans un solvant aromatique ou aprotique polaire organique.
